# EUROPEAN PATENT APPLICATION

(11) **EP 4 079 748 A1**
(43) Date of publication of application: **26.10.2022**
(21) Application number: 21170274.1
(22) Date of filing: 23.04.2021
(51) Int. Cl.: C07K 5/097, C07K 5/065, C07K 5/062, C07K 5/078, C07K 5/06

(54) **MODULATORS OF SORTILIN ACTIVITY**

(71) Applicant: Insusense ApS, 2100 Copenhagen (DK)
(72) Inventor: LITTLE, Paul Brian, 2100 Copenhagen (DK); CASES-THOMAS, Manuel Javier, 2100 Copenhagen (DK); KJØLBY, Mads Fuglsang, 2100 Copenhagen (DK); NYKJÆR, Anders, 2100 Copenhagen (DK)
(74) Representative: Gill Jennings & Every LLP

(57) **Abstract**

The present invention relates to compounds of formula (I), which are modulators of sortilin activity. The invention also relates to pharmaceutical compositions comprising these compounds and to the use of these compounds in the treatment or prevention of medical conditions where modulation of sortilin activity is beneficial.

SEQUENCE LISTING

<110>  Insusense ApS

<120>  COMPOUNDS

<130>  RMC00202EP

<160>  3

<170>  PatentIn version 3.5

<210>  1
<211>  831
<212>  PRT
<213>  Homo sapiens

<400>  1

<210>  2
<211>  830
<212>  PRT
<213>  Homo sapiens

<400>  2

Met Glu Arg Pro Trp Gly Ala Ala Asp Gly Leu Ser Arg Trp Pro His
1               5                   10                  15

Gly Leu Gly Leu Leu Leu Leu Leu Gln Leu Leu Pro Pro Ser Thr Leu
            20                  25                  30

Ser Gln Asp Arg Leu Asp Ala Pro Pro Pro Pro Ala Ala Pro Leu Pro
        35                  40                  45

Arg Trp Ser Gly Pro Ile Gly Val Ser Trp Gly Leu Arg Ala Ala Ala
    50                  55                  60

Ala Gly Gly Ala Phe Pro Arg Gly Gly Arg Trp Arg Arg Ser Ala Pro
65                  70                  75                  80

Gly Glu Asp Glu Glu Cys Gly Arg Val Arg Asp Phe Val Ala Lys Leu
                85                  90                  95

Ala Asn Asn Thr His Gln His Val Phe Asp Asp Leu Arg Gly Ser Val
            100                 105                 110

Ser Leu Ser Trp Val Gly Asp Ser Thr Gly Val Ile Leu Val Leu Thr
        115                 120                 125

Thr Phe His Val Pro Leu Val Ile Met Thr Phe Gly Gln Ser Lys Leu
    130                 135                 140

Tyr Arg Ser Glu Asp Tyr Gly Lys Asn Phe Lys Asp Ile Thr Asp Leu
145                 150                 155                 160

Ile Asn Asn Thr Phe Ile Arg Thr Glu Phe Gly Met Ala Ile Gly Pro
                165                 170                 175

Glu Asn Ser Gly Lys Val Val Leu Thr Ala Glu Val Ser Gly Gly Ser
            180                 185                 190

Arg Gly Gly Arg Ile Phe Arg Ser Ser Asp Phe Ala Lys Asn Phe Val
        195                 200                 205

Gln Thr Asp Leu Pro Phe His Pro Leu Thr Gln Met Met Tyr Ser Pro
    210                 215                 220

Gln Asn Ser Asp Tyr Leu Leu Ala Leu Ser Thr Glu Asn Gly Leu Trp
225                 230                 235                 240

Val Ser Lys Asn Phe Gly Gly Lys Trp Glu Glu Ile His Lys Ala Val
                245                 250                 255

Cys Leu Ala Lys Trp Gly Ser Asp Asn Thr Ile Phe Phe Thr Thr Tyr
            260                 265                 270

Ala Asn Gly Ser Cys Thr Asp Leu Gly Ala Leu Glu Leu Trp Arg Thr
        275                 280                 285

Ser Asp Leu Gly Lys Ser Phe Lys Thr Ile Gly Val Lys Ile Tyr Ser
    290                 295                 300

Phe Gly Leu Gly Gly Arg Phe Leu Phe Ala Ser Val Met Ala Asp Lys
305                 310                 315                 320

Asp Thr Thr Arg Arg Ile His Val Ser Thr Asp Gln Gly Asp Thr Trp

                325                 330                 335

Ser Met Ala Gln Leu Pro Ser Val Gly Gln Glu Gln Phe Tyr Ser Ile
            340                 345                 350

Leu Ala Ala Asn Asp Asp Met Val Phe Met His Val Asp Glu Pro Gly
        355                 360                 365

Asp Thr Gly Phe Gly Thr Ile Phe Thr Ser Asp Asp Arg Gly Ile Val
    370                 375                 380

Tyr Ser Lys Ser Leu Asp Arg His Leu Tyr Thr Thr Thr Gly Gly Glu
385                 390                 395                 400

Thr Asp Phe Thr Asn Val Thr Ser Leu Arg Gly Val Tyr Ile Thr Ser
                405                 410                 415

Val Leu Ser Glu Asp Asn Ser Ile Gln Thr Met Ile Thr Phe Asp Gln
            420                 425                 430

Gly Gly Arg Trp Thr His Leu Arg Lys Pro Glu Asn Ser Glu Cys Asp
        435                 440                 445

Ala Thr Ala Lys Asn Lys Asn Glu Cys Ser Leu His Ile His Ala Ser
    450                 455                 460

Tyr Ser Ile Ser Gln Lys Leu Asn Val Pro Met Ala Pro Leu Ser Glu
465                 470                 475                 480

Pro Asn Ala Val Gly Ile Val Ile Ala His Gly Ser Val Gly Asp Ala
                485                 490                 495

Ile Ser Val Met Val Pro Asp Val Tyr Ile Ser Asp Asp Gly Gly Tyr
            500                 505                 510

Ser Trp Thr Lys Met Leu Glu Gly Pro His Tyr Tyr Thr Ile Leu Asp
        515                 520                 525

Ser Gly Gly Ile Ile Val Ala Ile Glu His Ser Ser Arg Pro Ile Asn
    530                 535                 540

Val Ile Lys Phe Ser Thr Asp Glu Gly Gln Cys Trp Gln Thr Tyr Thr
545                 550                 555                 560

Phe Thr Arg Asp Pro Ile Tyr Phe Thr Gly Leu Ala Ser Glu Pro Gly
                565                 570                 575

Ala Arg Ser Met Asn Ile Ser Ile Trp Gly Phe Thr Glu Ser Phe Leu
            580                 585                 590

Thr Ser Gln Trp Val Ser Tyr Thr Ile Asp Phe Lys Asp Ile Leu Glu
        595                 600                 605

Arg Asn Cys Glu Glu Lys Asp Tyr Thr Ile Trp Leu Ala His Ser Thr
    610                 615                 620

Asp Pro Glu Asp Tyr Glu Asp Gly Cys Ile Leu Gly Tyr Lys Glu Gln
625                 630                 635                 640

Phe Leu Arg Leu Arg Lys Ser Ser Val Cys Gln Asn Gly Arg Asp Tyr
                645                 650                 655

Val Val Thr Lys Gln Pro Ser Ile Cys Leu Cys Ser Leu Glu Asp Phe
            660                 665                 670

Leu Cys Asp Phe Gly Tyr Tyr Arg Pro Glu Asn Asp Ser Lys Cys Val
        675                 680                 685

Glu Gln Pro Glu Leu Lys Gly His Asp Leu Glu Phe Cys Leu Tyr Gly
    690                 695                 700

Arg Glu Glu His Leu Thr Thr Asn Gly Tyr Arg Lys Ile Pro Gly Asp
705                 710                 715                 720

Lys Cys Gln Gly Gly Val Asn Pro Val Arg Glu Val Lys Asp Leu Lys
                725                 730                 735

Lys Lys Cys Thr Ser Asn Phe Leu Ser Pro Glu Lys Gln Asn Ser Lys
            740                 745                 750

Ser Asn Ser Val Pro Ile Ile Leu Ala Ile Val Gly Leu Met Leu Val
        755                 760                 765

Thr Val Val Ala Gly Val Leu Ile Val Lys Lys Tyr Val Cys Gly Gly
    770                 775                 780

Arg Phe Leu Val His Arg Tyr Ser Val Leu Gln Gln His Ala Glu Ala
785                 790                 795                 800

Asn Gly Val Asp Gly Val Asp Ala Leu Asp Thr Ala Ser His Thr Asn
                805                 810                 815

Lys Ser Gly Tyr His Asp Asp Ser Asp Glu Asp Leu Leu Glu
            820                 825                 830

<210>  3
<211>  694
<212>  PRT
<213>  Homo sapiens

<400>  3

Met Thr Phe Gly Gln Ser Lys Leu Tyr Arg Ser Glu Asp Tyr Gly Lys
1               5                   10                  15

Asn Phe Lys Asp Ile Thr Asp Leu Ile Asn Asn Thr Phe Ile Arg Thr
            20                  25                  30

Glu Phe Gly Met Ala Ile Gly Pro Glu Asn Ser Gly Lys Val Val Leu
        35                  40                  45

Thr Ala Glu Val Ser Gly Gly Ser Arg Gly Gly Arg Ile Phe Arg Ser
    50                  55                  60

Ser Asp Phe Ala Lys Asn Phe Val Gln Thr Asp Leu Pro Phe His Pro
65                  70                  75                  80

Leu Thr Gln Met Met Tyr Ser Pro Gln Asn Ser Asp Tyr Leu Leu Ala
                85                  90                  95

Leu Ser Thr Glu Asn Gly Leu Trp Val Ser Lys Asn Phe Gly Gly Lys
            100                 105                 110

Trp Glu Glu Ile His Lys Ala Val Cys Leu Ala Lys Trp Gly Ser Asp
        115                 120                 125

Asn Thr Ile Phe Phe Thr Thr Tyr Ala Asn Gly Ser Cys Thr Asp Leu
    130                 135                 140

Gly Ala Leu Glu Leu Trp Arg Thr Ser Asp Leu Gly Lys Ser Phe Lys
145                 150                 155                 160

Thr Ile Gly Val Lys Ile Tyr Ser Phe Gly Leu Gly Gly Arg Phe Leu
                165                 170                 175

Phe Ala Ser Val Met Ala Asp Lys Asp Thr Thr Arg Arg Ile His Val
            180                 185                 190

Ser Thr Asp Gln Gly Asp Thr Trp Ser Met Ala Gln Leu Pro Ser Val
        195                 200                 205

Gly Gln Glu Gln Phe Tyr Ser Ile Leu Ala Ala Asn Asp Asp Met Val
    210                 215                 220

Phe Met His Val Asp Glu Pro Gly Asp Thr Gly Phe Gly Thr Ile Phe
225                 230                 235                 240

Thr Ser Asp Asp Arg Gly Ile Val Tyr Ser Lys Ser Leu Asp Arg His
                245                 250                 255

Leu Tyr Thr Thr Thr Gly Gly Glu Thr Asp Phe Thr Asn Val Thr Ser
            260                 265                 270

Leu Arg Gly Val Tyr Ile Thr Ser Val Leu Ser Glu Asp Asn Ser Ile
        275                 280                 285

Gln Thr Met Ile Thr Phe Asp Gln Gly Gly Arg Trp Thr His Leu Arg
    290                 295                 300

Lys Pro Glu Asn Ser Glu Cys Asp Ala Thr Ala Lys Asn Lys Asn Glu
305                 310                 315                 320

Cys Ser Leu His Ile His Ala Ser Tyr Ser Ile Ser Gln Lys Leu Asn
                325                 330                 335

Val Pro Met Ala Pro Leu Ser Glu Pro Asn Ala Val Gly Ile Val Ile
            340                 345                 350

Ala His Gly Ser Val Gly Asp Ala Ile Ser Val Met Val Pro Asp Val
        355                 360                 365

Tyr Ile Ser Asp Asp Gly Gly Tyr Ser Trp Thr Lys Met Leu Glu Gly
    370                 375                 380

Pro His Tyr Tyr Thr Ile Leu Asp Ser Gly Gly Ile Ile Val Ala Ile
385                 390                 395                 400

Glu His Ser Ser Arg Pro Ile Asn Val Ile Lys Phe Ser Thr Asp Glu
                405                 410                 415

Gly Gln Cys Trp Gln Thr Tyr Thr Phe Thr Arg Asp Pro Ile Tyr Phe
            420                 425                 430

Thr Gly Leu Ala Ser Glu Pro Gly Ala Arg Ser Met Asn Ile Ser Ile
        435                 440                 445

Trp Gly Phe Thr Glu Ser Phe Leu Thr Ser Gln Trp Val Ser Tyr Thr
    450                 455                 460

Ile Asp Phe Lys Asp Ile Leu Glu Arg Asn Cys Glu Glu Lys Asp Tyr
465                 470                 475                 480

Thr Ile Trp Leu Ala His Ser Thr Asp Pro Glu Asp Tyr Glu Asp Gly
                485                 490                 495

Cys Ile Leu Gly Tyr Lys Glu Gln Phe Leu Arg Leu Arg Lys Ser Ser
            500                 505                 510

Val Cys Gln Asn Gly Arg Asp Tyr Val Val Thr Lys Gln Pro Ser Ile
        515                 520                 525

Cys Leu Cys Ser Leu Glu Asp Phe Leu Cys Asp Phe Gly Tyr Tyr Arg
    530                 535                 540

Pro Glu Asn Asp Ser Lys Cys Val Glu Gln Pro Glu Leu Lys Gly His
545                 550                 555                 560

Asp Leu Glu Phe Cys Leu Tyr Gly Arg Glu Glu His Leu Thr Thr Asn
                565                 570                 575

Gly Tyr Arg Lys Ile Pro Gly Asp Lys Cys Gln Gly Gly Val Asn Pro
            580                 585                 590

Val Arg Glu Val Lys Asp Leu Lys Lys Lys Cys Thr Ser Asn Phe Leu
        595                 600                 605

Ser Pro Glu Lys Gln Asn Ser Lys Ser Asn Ser Val Pro Ile Ile Leu
    610                 615                 620

Ala Ile Val Gly Leu Met Leu Val Thr Val Val Ala Gly Val Leu Ile
625                 630                 635                 640

Val Lys Lys Tyr Val Cys Gly Gly Arg Phe Leu Val His Arg Tyr Ser
                645                 650                 655

Val Leu Gln Gln His Ala Glu Ala Asn Gly Val Asp Gly Val Asp Ala
            660                 665                 670

Leu Asp Thr Ala Ser His Thr Asn Lys Ser Gly Tyr His Asp Asp Ser
        675                 680                 685

Asp Glu Asp Leu Leu Glu
    690

## Description

### FIELD OF THE INVENTION

The present invention relates to compounds of formula (I), which are modulators of sortilin activity. The invention also relates to pharmaceutical compositions comprising these compounds and to the use of these compounds in the treatment or prevention of medical conditions where modulation of sortilin activity is beneficial. Such medical conditions include a neurodegenerative disorder, an inflammatory disorder, a cancer, pain, diabetes mellitus, diabetic retinopathy, glaucoma, uveitis, cardiovascular disease, hereditary eye conditions or hearing loss.

### BACKGROUND

Sortilin (encoded by *SORT1*) is a type 1 membrane receptor in the vacuolar protein sorting 10 protein (VPS10P) family of sorting receptors, and is abundantly expressed in the central nervous system, the inner ear, and in some peripheral tissues involved in metabolic control^{l,2,3,4}. Sortilin has an amino acid sequence according to SEQ ID NO: 1 and comprises a signal peptide, a propeptide, the Vps10p domain, a 10cc domain (10CCa + 10CCb), a transmembrane domain and a large cytoplasmic tail. The luminal domain of sortilin has 6 potential N-linked glycosylation sites, whilst the cytoplasmic tail enables for the recruitment of various adapter proteins.

Sortilin binds to a vast number of ligands and membrane receptors and as a result engages in functions known to be important in cellular signalling and sorting. For example, sortilin is involved in signalling by proneurotrophins: the proforms of nerve growth factor (proNGF), brain derived neurotrophic factor (proBDNF), and neurotrophin-3 (proNT3), respectively. In complex with the protein p75NTR (p75 neurotrophin receptor), sortilin has been reported to form the receptor for proneurotrophin-mediated apoptotic effects leading to degeneration and cell death in cellular and animal models^{5,6,7}.

Previous work has suggested a role for sortilin in cellular sorting and signalling associated with diseases such as diabetes and obesity (Huang et al 2013 Mol Biol Cell Oct;24(19):3115-22)⁸. Sortilin facilitates translocation of GLUT4 to the plasma membrane and rescues it from degradation in the lysosomes (Pan et al Mol Biol Cell. 2017 Jun 15;28(12):1667-1675)⁹. Sortilin levels have been shown to be modulated by the level of inflammation associated with these diseases. The pro-inflammatory cytokine, TNFα, reduces both mRNA levels and protein levels of sortilin in cultured mouse and human adipocytes, as well as *in vivo* when injected into mice (Kaddai et al. Diabetologia 52: 932-40, 2009)¹⁰. Sortilin can also influence cytokine secretion: targeting sortilin in immune cells has been proposed to attenuate inflammation and reduce atherosclerosis disease progression (Mortensen et al. J Clin Invest 124(12):5317-22, 2014)¹¹. Additionally, US 2016/0331746 describes various scaffolds of small molecules capable of binding to the active site of sortilin. Sortilin is involved in the regulation of glucose uptake (Shi & Kandror. Developmental Cell 9:99-108, 2005)¹² and the development of lipid disorder diseases (Gao et al. DNA and Cell Biology 36(12):1050-61, 2017)¹³.

Further, plasma sortilin levels have been reported to be a potential biomarker for identifying patients with either coronary heart disease or diabetes mellitus (Oh et al. Cardiovascular Diabetology 16:92, 2017)¹⁴. Patients that showed increased sortilin levels within their plasma, and therefore identifiable as suffering from the above conditions, also displayed enhanced glucose levels suggesting sortilin as a therapeutic target for treating these conditions.

In view of the above, there is an unmet need for new compounds that may be used in the treatment and prevention of medical conditions in which modulation of sortilin is beneficial, such as a neurodegenerative disorder, an inflammatory disorder, a cancer, pain, diabetes mellitus, diabetic retinopathy, glaucoma, uveitis, cardiovascular disease, hereditary eye conditions or hearing loss. The neurodegenerative disorder may be selected from frontotemporal dementia, Alzheimer's disease, Parkinson's disease and spinal cord injury; the inflammatory disorder may be selected from inflammatory diseases and neuroinflammation; the cancer may be selected from breast cancer, lung cancer, ovarian cancer, prostate cancer, thyroid cancer, pancreatic cancer, glioblastoma and colorectal cancer; and the hearing loss may be selected from noise-induced hearing loss, ototoxicity induced hearing loss, age-induced hearing loss, idiopathic hearing loss, tinnitus and sudden hearing loss.

### DESCRIPTION OF THE FIGURES

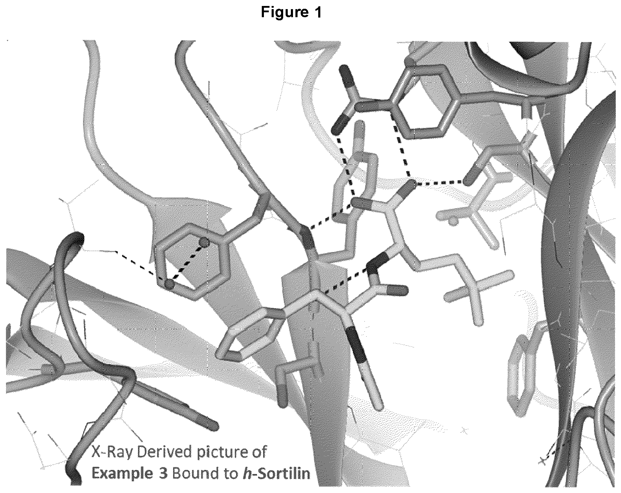

Figure 1 is an X-Ray Derived picture of Example 3 bound to *h*-Sortilin.

### DISCLOSURE OF THE INVENTION

In a first aspect, the present invention provides a compound of formula (I) 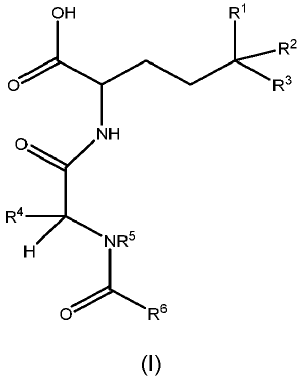
or a pharmaceutically acceptable salt, solvate, hydrate, tautomer, optical isomer, N-oxide, and/or prodrug thereof; wherein
R¹, R² and R³ are each independently selected from the group consisting of halo, H, (C₁-C₄)alkyl, halo-(C₁-C₄)alkyl, (C₂-C₄)alkenyl, and halo-(C₂-C₄)alkenyl;
R⁴ is selected from the group consisting of H, (C₁-C₁₀)alkyl, halo-(C₁-C₁₀)alkyl, (C₂-C₁₀alkenyl, halo-(C₂-C₁₀)alkenyl, (C₃-C₈)aryl, halo-(C₃-C₈)aryl, (C₃-C₈)heteroaryl, halo-(C₃-C₈)heteroaryl, (C₁-C₆)-alkylene-(C₃-C₂₀)-aryl, (C₁-C₆)-alkylene-(C₃-C₂₀)-heteroaryl, (C₁-C₆)-alkylene-(3- to 10- membered-heterocyclic ring);
   wherein the aryl group in (C₁-C₆)-alkylene-(C₃-C₂₀)-aryl, the heteroaryl group in (C₁-C₆)-alkylene-(C₃-C₂₀)-heteroaryl or the heterocyclic ring in (C₁-C₆)-alkylene-(3- to 8- membered heterocyclic ring) is optionally substituted with one or more substituents independently selected from halo, H, -OH, (C₁-C₄)alkyl, halo-(C₁-C₄)alkyl, (C₁-C₄)alkoxy and halo-(C₁-C₄)alkoxy;
R⁵ is selected from the group consisting of H, (C₁-C₄)alkyl, halo-(C₁-C₄)alkyl, (C₂-C₄)alkenyl and halo-(C₁-C₄)alkenyl; and
R⁶ is selected from the group consisting of (C₁-C₆)alkyl, halo-(C₁-C₆)alkyl, (C₂-C₆)alkenyl, halo-(C₂-C₆)alkenyl, (C₃-C₈)aryl, halo-(C₃-C₈)aryl, (C₃-C₈)heteroaryl and halo-(C₃-C₈)heteroaryl.

It has been surprisingly found that compounds of formula (I) inhibit or antagonise sortilin and therefore may be useful in conditions where sortilin inhibition is beneficial. Such conditions include a neurodegenerative disorder, an inflammatory disorder, a cancer, pain, diabetes mellitus, diabetic retinopathy, glaucoma, uveitis, cardiovascular disease, hereditary eye conditions or hearing loss. The neurodegenerative disorder may be selected from frontotemporal dementia, Alzheimer's disease, Parkinson's disease and spinal cord injury; the inflammatory disorder may be selected from inflammatory diseases and neuroinflammation; the cancer may be selected from breast cancer, lung cancer, ovarian cancer, prostate cancer, thyroid cancer, pancreatic cancer, glioblastoma and colorectal cancer; and the hearing loss may be selected from noise-induced hearing loss, ototoxicity induced hearing loss, age-induced hearing loss, idiopathic hearing loss, tinnitus and sudden hearing loss..

As used herein, the term "sortilin" may refer to full length sortilin (also referred to as immature sortilin), comprising a signal peptide, a propeptide, a Vps10p domain, a 10CC domain, a transmembrane domain and a large cytoplasmic tail, having an amino acid sequence according to SEQ ID NO: 1 or SEQ ID NO: 2, or it may refer to mature sortilin, comprising a Vps10p domain, a 10CC domain, a transmembrane domain and a large cytoplasmic tail, having an amino acid sequence according to SEQ ID NO: 3, or a naturally occurring fragment, homologue or variant thereof. The term "sortilin" or "sortilin molecule" are used interchangeably herein. It is understood that the sortilin is capable of interacting with a pro-neurotrophin molecule to form a sortilin/pro-neurotrophin complex. This sortilin/pro-neurotrophin complex may or may not be capable of interacting with a p75NTR molecule to form a trimeric complex comprising sortilin, pro-neurotrophin and p75NTR. It is understood that this trimeric complex may be responsible for adverse biological responses, such as stimulating apoptosis in retinal and ganglion cells, and controlling growth cone retraction of projecting axons.

As used herein, the term "pro-neurotrophin" refers to the larger precursors of neurotrophins, which undergo proteolytic cleavage to yield the mature form of the neurotrophin. Neurotrophins are a family of proteins that induce the survival, development and function of neurons, and are commonly referred to as growth factors. Pro-neurotrophins are biologically active and have distinct roles compared to their neurotrophin counterparts, such as induction of apoptosis. Examples of pro-neurotrophins include proNGF, proBDNF, proNT3 and proNT4. Pro-neurotrophins may also control synaptic plasticity. Whereas mature neurotrophins induce synaptic strength, in their proforms they may weaken synapses,

The compounds of the invention may be sortilin inhibitors or antagonists. As used herein, the term "sortilin antagonist" refers to a substance that interferes with, blocks, or otherwise attenuates the effect of, a sortilin protein binding to a pro-neurotrophin (e.g., proNGF, proNT3, proBDNF) and preventing the formation of the trimeric complex between sortilin, p75NTR and the pro-neurotrophin. The term "sortilin antagonist" also includes a substance or agent that interferes with the formation of a high affinity trimeric complex. In the latter scenario, it is recognised that a trimeric complex may be formed in that sortilin can bind to p75NTR (but not proNGF) and p75NTR can simultaneously bind the NGF domain of proNGF. However, the resulting trimeric complex may be of lower affinity for its receptor and as a result have significantly reduced capacity to stimulate apoptosis via the mechanism described above. Skeldal et al (J. Biol. Chem. 2012 Dec 21;287(52):43798-809)¹⁵ demonstrated that the apoptotic function of the trimeric complex is abolished when sortilin is devoid in its intracellular domain. The term "sortilin antagonist" also includes a substance or agent that interferes with, blocks, or otherwise attenuates the effect of, a sortilin protein interacting with p75NTR. This interaction may be completely prevented, in which case the trimeric complex is prevented from forming, or only partially prevented, in which case the trimeric complex may be formed but may have reduced biological potency. Skeldal et al showed that complex formation between sortilin and p75NTR relies on contact points in the extracellular domains of the receptors and that the interaction critically depends on an extracellular juxtamembrane 23-amino acid sequence of p75NTR. Thus, the sortilin antagonist may interfere with this 23-amino acid sequence or proximal sequences in the molecules.

In the first aspect of the invention, it is preferred that R¹, R² and R³ are each independently selected from the group consisting of halo, (C₁-C₂)alkyl and halo-(C₁-C₂)alkyl.

In a preferred aspect of the invention, R¹, R² and R³ are each independently selected from F, CH₃ and CF₃. Most preferably, R¹, R² and R³ are the same. For example, in an exemplary compound of the invention, R¹, R² and R³ may each be F, CH₃ or CF₃.

In another preferred aspect of the invention, R⁴ is selected from the group consisting of H, (C₁-C₆)alkyl, halo-(C₁-C₆)alkyl, (C₃-C₁₀)aryl, (C₁-C₃)-alkylene-(C₃-C₁₀)-aryl, (C₁-C₃)-alkylene-(C₃-C₂₀)-heteroaryl and (C₁-C₃)-alkylene-(3- to 10-membered-heterocyclic ring). The aryl group in (C₁-C₃)-alkylene-(C₃-C₁₀)-aryl, the heteroaryl group in (C₁-C₃)-alkylene-(C₃-C₁₀)-heteroaryl or the heterocyclic ring in (C₁-C₃)-alkylene-(3- to 8- membered heterocyclic ring) is optionally substituted with one or more substituents independently selected from halo, -OH, (C₁-C₄)alkyl, halo-(C₁-C₄)alkyl, (C₁-C₄)alkoxy and halo-(C₁-C₄)alkoxy.

The alkyl, haloalkyl, alkenyl, haloalkenyl groups and the alkyl, haloalkyl, alkoxy and haloalkoxy substituents may be linear or branched.

The substituent may be attached at any position of the aryl, heteroaryl or heterocyclic ring. The one or more substituents may be attached to a carbon atom, heteroatom or combinations thereof. Preferably, there are no substituents or between one to three substituents.

The heteroaryl or heterocyclic ring may comprise one, two or more heteroatoms. Preferably, the heteroaryl or heterocyclic ring comprises one or two heteroatoms. The heteroatom may be selected from N, S or O. In groups with more than one heteroatom present, the heteroatoms may be the same or they may be different.

The heterocyclic ring is may be aliphatic. It may be monocyclic, bicyclic or tricyclic. Preferably, the heterocyclic ring is monocyclic or bicyclic. Preferably, the heterocyclic ring has between 5-7 members.

The aryl and heteroaryl groups may also be monocyclic, bicyclic or tricyclic. Preferably, monocyclic or bicyclic. Preferably, the aryl and heteroaryl groups have a ring size of between 5-9 members.

In some preferred embodiments, R⁴ is selected from the group consisting of:

(i) H

(ii) CH₃

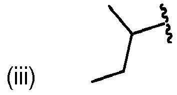

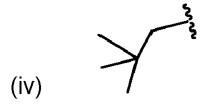

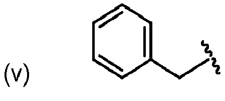

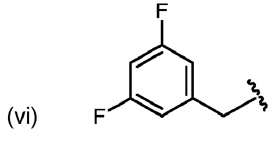

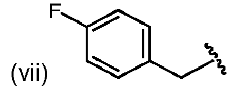

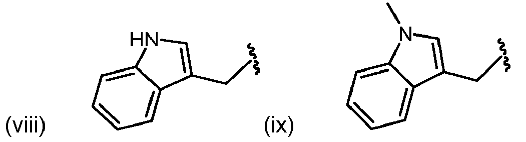

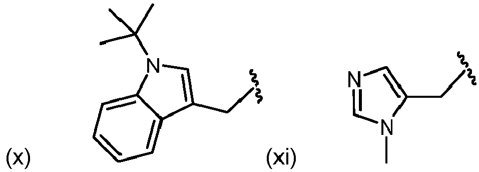

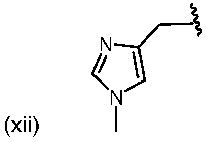

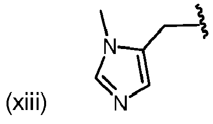

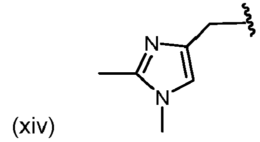

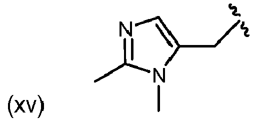

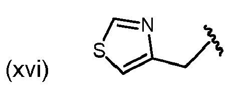

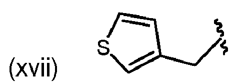

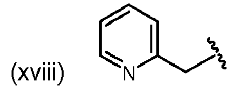

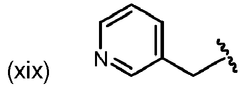

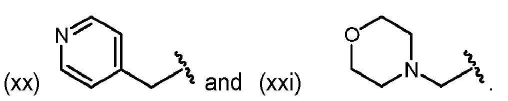

The chirality of the carbon of formula (I) attached to R⁴ is either (R) or (S). Preferably, when groups (i)-(xii) and (ix)-(xx) above are included in formula (I), the chirality of this carbon is (S), and group (xiii) may be (S) or (R).

In another preferred aspect of the invention, R⁵ is selected from the group consisting of H, (C₁-C₃)-alkyl and (C₁-C₃)haloalkyl.

Preferably, R⁵ is selected from the group consisting of H and CH₃.

In another preferred aspect of the invention, R⁶ is selected from the group consisting of (C₁-C₄)alkyl, halo-(C₁-C₄)alkyl, (C₃-C₈)heteroaryl, and halo-(C₃-C₈)heteroaryl.

Most preferably, R⁶ is selected from the group consisting of CH₃, pyrazine and morpholine.

Particular compounds of the invention are those listed below.
(S)-2-(2-acetamidoacetamido)-5,5-dimethylhexanoic acid;
(S)-5,5-dimethyl-2-(2-(pyrazine-2-carboxamido)acetamido)hexanoic acid;
(S)-2-((S)-2-acetamido-3-phenylpropanamido)-5,5-dimethylhexanoic acid;
(2S)-5,5-dimethyl-2-{2-[(pyrazin-2-yl)formamido]acetamido}hexanoic acid;
(2S)-2-[(2S,3S)-2-acetamido-3-methylpentanamido]-5,5-dimethylhexanoic acid;
(2S)-5,5-dimethyl-2-[(2S,3S)-3-methyl-2-[(pyrazin-2-yl)formamido]pentanamido]hexanoic acid;
(2S)-5,5,5-trifluoro-2-[(2S,3S)-3-methyl-2-[(pyrazin-2-yl)formamido]pentanamido]pentanoic acid;
(2S)-2-[(2S)-2-acetamido-3-phenylpropanamido]-5,5-dimethylhexanoic acid;
(2S)-2-[(2S)-2-acetamido-3-(4-fluorophenyl)propanamido]-5,5-dimethylhexanoic acid;
(2S)-2-[(2S)-3-(3,5-difluorophenyl)-2-acetamidopropanamido]-5,5-dimethylhexanoic acid;
(2S)-2-[(2S)-2-acetamido-3-(1H-indol-3-yl)propanamido]-5,5-dimethylhexanoic acid;
(2S)-2-[(2S)-2-acetamido-3-(1-methyl-1H-imidazol-5-yl)propanamido]-5,5-dimethylhexanoic acid;
(S)-5,5-dimethyl-2-((S)-3-phenyl-2-(pyrazine-2-carboxamido)propanamido)hexanoic acid;
(2S)-2-[(2S)-2-acetamido-3-(1-methyl-1H-imidazol-4-yl)propanamido]-5,5-dimethylhexanoic acid;
(2S)-5,5-dimethyl-2-[(2S,3S)-3-methyl-2-{[(3S)-morpholin-3-yl]formamido}pentanamido]hexanoic acid;
(2S)-5,5-dimethyl-2-[(2S,3S)-3-methyl-2-{[(3R)-morpholin-3-yl]formamido}pentanamido]hexanoic acid;
(2S)-5,5-dimethyl-2-[(2S,3S)-3-methyl-2-{[(2S)-morpholin-2-yl]formamido}pentanamido]hexanoic acid;
(2S)-5,5-dimethyl-2-[(2S,3S)-3-methyl-2-{[(2R)-morpholin-2-yl]formamido}pentanamido]hexanoic acid;
(2S)-5,5-dimethyl-2-[(2S)-3-(1-methyl-1H-imidazol-4-yl)-2-(N-methylacetamido)propanamido]hexanoic acid;
(2S)-5,5-dimethyl-2-[(2S)-3-(1-methyl-1H-imidazol-5-yl)-2-(N-methylacetamido)propanamido]hexanoic acid;
(2S)-5,5-dimethyl-2-[(2S)-3-(1-methyl-1H-imidazol-4-yl)-2-(2-oxopyrrolidin-1-yl)propanamido]hexanoic acid;
(2S) - 2 - [(2S) - 3 - (1,2 - dimethyl - 1H - imidazol - 5 - yl) - 2 - (N - methylacetamido)propanamido] - 5,5 - dimethylhexanoic acid;
(2S) - 2 - [(2S) - 3 - (1,2 - dimethyl - 1H - imidazol - 4 - yl) - 2 - (N - methylacetamido)propanamido] - 5,5 - dimethylhexanoic acid;
(2S) - 2 - [(2R) - 2 - acetamido - 3 - (1H - indol - 3 - yl)propanamido] - 5,5 - dimethylhexanoic acid;
(2S) - 2 - [(2S) - 2 - acetamido - 3 - (1,3 - thiazol - 4 - yl)propanamido] - 5,5 - dimethylhexanoic acid;
(2S) - 2 - [(2S) - 2 - acetamidopropanamido] - 5,5 - dimethylhexanoic acid;
(2S) - 2 - [(2S) - 2 - acetamido - 3 - (thiophen - 3 - yl)propanamido] - 5,5 - dimethylhexanoic acid;
(2S) - 2 - [(2S) - 2 - acetamido - 3 - (pyridin - 2 - yl)propanamido] - 5,5 - dimethylhexanoic acid;
(2S) - 2 - [(2S) - 2 - acetamido - 3 - (pyridin - 3 - yl)propanamido] - 5,5 - dimethylhexanoic acid;
(2S) - 2 - [(2S) - 2 - acetamido - 3 - (pyridin - 4 - yl)propanamido] - 5,5 - dimethylhexanoic acid;
(2S) - 2 - [(2S) - 2 - acetamido - 3 - (morpholin - 4 - yl)propanamido] - 5,5 - dimethylhexanoic acid; and
(2S) - 2 - [(2S) - 2 - acetamido - 3 - (1 - methyl - 1H - indol - 3 - yl)propanamido] - 5,5 - dimethylhexanoic acid.

The compounds of formula (I) are intended for use in the treatment or prevention of a neurodegenerative disorder, an inflammatory disorder, a cancer, pain, diabetes mellitus, diabetic retinopathy, glaucoma, uveitis, cardiovascular disease, hereditary eye conditions or hearing loss.

Preferably the neurodegenerative disorder is selected from frontotemporal dementia, Alzheimer's disease, Parkinson's disease and spinal cord injury.

Preferably the inflammatory disorder may be selected from inflammatory diseases and neuroinflammation;

Preferably the cancer is selected from breast cancer, lung cancer, ovarian cancer, prostate cancer, thyroid cancer, pancreatic cancer, glioblastoma and colorectal cancer.

Preferably the hearing loss is selected from noise-induced hearing loss, ototoxicity induced hearing loss, age-induced hearing loss, idiopathic hearing loss, tinnitus and sudden hearing loss.

Thus, in an embodiment, the compounds for use according to the invention may disrupt interaction between a sortilin molecule and a pro-neurotrophin molecule, or disrupt the interaction between a sortilin molecule and a p75NTR molecule. Said sortilin molecule may be mature sortilin.

Preferably, the compounds of the present invention are sortilin inhibitors. As used herein, the term "sortilin inhibitor" refers to a compound that binds to a sortilin protein, thereby preventing it from binding to a pro-neurotrophin or a p75NTR molecule and preventing the formation of the aforementioned trimeric complex, or resulting in the formation of a trimeric complex that is less active or inactive.

Preferably, the compounds of the present invention prevent the protein-protein interaction between a sortilin molecule and a pro-neurotrophin or a p75NTR molecule, further preventing the formation of the apoptotic trimeric complex usually formed between sortilin, pro-neurotrophin and the p75NTR receptor, or resulting in the formation of a low affinity trimeric complex, which is biologically less active or inactive or has minimal activity.

Thus, the compound may bind to sortilin, a pro-neutrophin or a p75NTR molecule. The antagonistic action may be due to direct blocking of protein-protein interaction or it could be by steric hindrance when bound at a site of one of these proteins apart from the binding site.

According to a second aspect of the invention, there is provided a pharmaceutical composition comprising a compound according to the first aspect of the invention and one or more pharmaceutically acceptable carriers, excipients, and/or diluents.

In a third aspect of the invention, there is provided a compound according to the first aspect of the invention, or a pharmaceutical composition according to the second aspect of the invention for use in therapy.

According to a fourth aspect of the invention, there is provided a compound according to the first aspect of the invention, or a pharmaceutical composition according to the second aspect of the invention for use in the treatment or prevention of a a neurodegenerative disorder, an inflammatory disorder, a cancer, pain, diabetes mellitus, diabetic retinopathy, glaucoma, uveitis, cardiovascular disease, hereditary eye conditions or hearing loss.

Preferably, the neurodegenerative disorder is selected from frontotemporal dementia, Alzheimer's disease, Parkinson's disease and spinal cord injury.

Preferably, the hearing loss is selected from noise-induced hearing loss, ototoxicity induced hearing loss, age-induced hearing loss, idiopathic hearing loss, tinnitus and sudden hearing loss.

Preferably, the cancer is selected from breast cancer, lung cancer, ovarian cancer, prostate cancer, thyroid cancer, pancreatic cancer, glioblastoma, and colorectal cancer.

Preferably the cardiovascular disease is selected from atherosclerosis, cardiomyopathy, heard attack, arrhythmias, and coronary artery disease.

According to a fifth aspect of the invention, there is provided the use of the compound according to the first aspect of the invention for the manufacture of a medicament for the treatment or prevention of a neurodegenerative disorder, an inflammatory disorder, a cancer, pain, diabetes mellitus, diabetic retinopathy, glaucoma, uveitis, cardiovascular disease, hereditary eye conditions or hearing loss.

According to a sixth aspect of the invention, there is provided a method for the treatment or prevention of a disease or condition responsive to sortilin modulation comprising administering a therapeutically effective amount of the compound according to the first aspect of the invention or the pharmaceutical composition according the second aspect of the invention.

The compounds of the invention may include isotopically-labelled and/or isotopically-enriched forms of the compounds. The compounds of the invention herein may contain unnatural proportions of atomic isotopes at one or more of the atoms that constitute such compounds. Examples of isotopes that can be incorporated into the disclosed compounds include isotopes of hydrogen, carbon, nitrogen, oxygen, phosphorus, sulfur, chlorine, such as ²H, ³H, ¹¹C, ¹³C, ¹⁴C, ¹³N, ¹⁵O, ¹⁷O, ³²P, ³⁵S, ¹⁸F, ³⁶Cl.

The compounds of the invention may be used as such or, where appropriate, as pharmacologically acceptable salts (acid or base addition salts) thereof. The pharmacologically acceptable addition salts mentioned below are meant to comprise the therapeutically active non-toxic acid and base addition salt forms that the compounds are able to form. Compounds that have basic properties can be converted to their pharmaceutically acceptable acid addition salts by treating the base form with an appropriate acid. Exemplary acids include inorganic acids, such as hydrogen chloride, hydrogen bromide, hydrogen iodide, sulphuric acid, phosphoric acid; and organic acids such as formic acid, acetic acid, propanoic acid, hydroxyacetic acid, lactic acid, pyruvic acid, glycolic acid, maleic acid, malonic acid, oxalic acid, benzenesulphonic acid, toluenesulphonic acid, methanesulphonic acid, trifluoroacetic acid, fumaric acid, succinic acid, malic acid, tartaric acid, citric acid, salicylic acid, p-aminosalicylic acid, pamoic acid, benzoic acid, ascorbic acid and the like. Compounds that have acidic properties can be converted to their pharmaceutically acceptable basic addition salts by treating the acid form with an appropriate base. Exemplary base addition salt forms are the sodium, potassium, calcium salts, and salts with pharmaceutically acceptable amines such as, for example, ammonia, alkylamines, benzathine, and amino acids, such as, e.g. arginine and lysine. The term addition salt as used herein also comprises solvates which the compounds and salts thereof are able to form, such as, for example, hydrates, alcoholates and the like.

Throughout the present disclosure, a given chemical formula or name shall also encompass all pharmaceutically acceptable salts, solvates, hydrates, N-oxides, and/or prodrug forms thereof. It is to be understood that the compounds of the invention include any and all hydrates and/or solvates of the compound formulas.

It is appreciated that certain functional groups, such as the hydroxy, amino, and like groups form complexes and/or coordination compounds with water and/or various solvents, in the various physical forms of the compounds. Accordingly, the above formulas are to be understood to include and represent those various hydrates and/or solvates.

Compounds of the invention also include tautomeric forms. Tautomeric forms result from the swapping of a single bond with an adjacent double bond together with the concomitant migration of a proton. Tautomeric forms include prototropic tautomers which are isomeric protonation states having the same empirical formula and total charge. Example prototropic tautomers include ketone - enol pairs, amide - imidic acid pairs, lactam - lactim pairs, amide - imidic acid pairs, enamine - imine pairs, and annular forms where a proton can occupy two or more positions of a heterocyclic system, for example, 1H- and 3H-imidazole, 1H, 2Hand 4H- 1,2,4-triazole, 1H- and 2H- isoindole, and 1H- and 2H-pyrazole. Tautomeric forms can be in equilibrium or sterically locked into one form by appropriate substitution.

The compounds described herein can be asymmetric (e.g. having one or more stereocenters). All stereoisomers, such as enantiomers and diastereomers, are intended unless otherwise indicated. Compounds of the present invention that contain asymmetrically substituted carbon atoms can be isolated in optically active or racemic forms. Methods on how to prepare optically active forms from optically active starting materials are known in the art, such as by resolution of racemic mixtures or by stereoselective synthesis. Many geometric isomers of olefins, C=N double bonds, and the like can also be present in the compounds described herein, and all such stable isomers are contemplated in the present invention. Cis- and trans-geometric isomers of the compounds of the present invention are described and may be isolated as a mixture of isomers or as separated isomeric forms.

In the case of the compounds which contain an asymmetric carbon atom, the invention relates to the D form, the L form, and D,L mixtures and also, where more than one asymmetric carbon atom is present, to the diastereomeric forms. Those compounds of the invention which contain asymmetric carbon atoms, and which as a rule accrue as racemates, can be separated into the optically active isomers in a known manner, for example using an optically active acid. However, it is also possible to use an optically active starting substance from the outset, with a corresponding optically active or diastereomeric compound then being obtained as the end product.

The term "prodrugs" refers to compounds that may be converted under physiological conditions or by solvolysis to a biologically active compound of the invention. A prodrug may be inactive when administered to a subject in need thereof, but is converted in vivo to an active compound of the invention. Prodrugs are typically rapidly transformed in vivo to yield the parent compound of the invention, e.g. by hydrolysis in the blood. The prodrug compound usually offers advantages of solubility, tissue compatibility or delayed release in a mammalian organism (see Silverman, R. B., The Organic Chemistry of Drug Design and Drug Action, 2nd Ed., Elsevier Academic Press (2004), page 498 to 549). Prodrugs of a compound of the invention may be prepared by modifying functional groups, such as a hydroxy, amino or mercapto groups, present in a compound of the invention in such a way that the modifications are cleaved, either in routine manipulation or in vivo, to the parent compound of the invention. Examples of prodrugs include, but are not limited to, acetate, formate and succinate derivatives of hydroxy functional groups or phenyl carbamate derivatives of amino functional groups.

The term "treatment" as used herein may include prophylaxis of the named disorder or condition, or amelioration or elimination of the disorder once it has been established. The term "prevention" refers to prophylaxis of the named disorder or condition.

Methods delineated herein include those wherein the subject is identified as in need of a particular stated treatment. Identifying a subject in need of such treatment can be in the judgment of a subject or a health care professional and can be subjective (e.g. opinion) or objective (e.g. measurable by a test or diagnostic method).

In other aspects, the methods herein include those further comprising monitoring subject response to the treatment administrations. Such monitoring may include periodic imaging or sampling of subject tissue, fluids, specimens, cells, proteins, chemical markers, genetic materials, Etc. as markers or indicators of the treatment regimen. In other methods, the subject is pre-screened or identified as in need of such treatment by assessment for a relevant marker or indicator of suitability for such treatment.

The invention provides a method of monitoring treatment progress. The method includes the step of determining a level of diagnostic marker (Marker) (e.g. any target or cell type delineated herein modulated by a compound herein) or diagnostic measurement (e.g., screen, assay) in a subject suffering from or susceptible to a disorder or symptoms thereof delineated herein, in which the subject has been administered a therapeutic amount of a compound herein sufficient to treat the disease or symptoms thereof. The level of Marker determined in the method can be compared to known levels of Marker in either healthy normal controls or in other afflicted patients to establish the subject's disease status. In preferred embodiments, a second level of Marker in the subject is determined at a time point later than the determination of the first level, and the two levels are compared to monitor the course of disease or the efficacy of the therapy. In certain preferred embodiments, a pre-treatment level of Marker in the subject is determined prior to beginning treatment according to this invention; this pretreatment level of Marker can then be compared to the level of Marker in the subject after the treatment commences, to determine the efficacy of the treatment.

A level of Marker or Marker activity in a subject may be determined at least once. Comparison of Marker levels, e.g., to another measurement of Marker level obtained previously or subsequently from the same patient, another patient, or a normal subject, may be useful in determining whether therapy according to the invention is having the desired effect, and thereby permitting adjustment of dosage levels as appropriate. Determination of Marker levels may be performed using any suitable sampling/expression assay method known in the art or described herein. Preferably, a tissue or fluid sample is first removed from a subject. Examples of suitable samples include blood, urine, tissue, mouth or cheek cells, and hair samples containing roots. Other suitable samples would be known to the person skilled in the art. Determination of protein levels and/or mRNA levels (e.g., Marker levels) in the sample can be performed using any suitable technique known in the art, including, but not limited to, enzyme immunoassay, ELISA, radiolabeling/assay techniques, blotting/chemiluminescence methods, real-time PCR, and the like.

For clinical use, the compounds disclosed herein are formulated into pharmaceutical compositions (or formulations) for various modes of administration. It will be appreciated that compounds of the invention may be administered together with a physiologically acceptable carrier, excipient, and/or diluent (i.e. one, two, or all three of these). The pharmaceutical compositions disclosed herein may be administered by any suitable route, preferably by oral, rectal, nasal, topical (including ophthalmic, buccal and sublingual), sublingual, transdermal, intrathecal, transmucosal or parenteral (including subcutaneous, intramuscular, intravenous and intradermal) administration. Other formulations may conveniently be presented in unit dosage form, e.g., tablets and sustained release capsules, and in liposomes, and may be prepared by any methods well known in the art of pharmacy. Pharmaceutical formulations are usually prepared by mixing the active substance, or a pharmaceutically acceptable salt thereof, with conventional pharmaceutically acceptable carriers, diluents or excipients. Examples of excipients are water, gelatin, gum arabicum, lactose, microcrystalline cellulose, starch, sodium starch glycolate, calcium hydrogen phosphate, magnesium stearate, talcum, colloidal silicon dioxide, and the like. Such formulations may also contain other pharmacologically active agents, and conventional additives, such as stabilizers, wetting agents, emulsifiers, flavouring agents, buffers, and the like. Usually, the amount of active compounds is between 0.1-95% by weight of the preparation, preferably between 0.2-20% by weight in preparations for parenteral use and more preferably between 1-50% by weight in preparations for oral administration. The formulations can be further prepared by known methods such as granulation, compression, microencapsulation, spray coating, Etc. The formulations may be prepared by conventional methods in the dosage form of tablets, capsules, granules, powders, syrups, suspensions, suppositories or injections. Liquid formulations may be prepared by dissolving or suspending the active substance in water or other suitable vehicles. Tablets and granules may be coated in a conventional manner. To maintain therapeutically effective plasma concentrations for extended periods of time, compounds disclosed herein may be incorporated into slow release formulations.

The dose level and frequency of dosage of the specific compound will vary depending on a variety of factors including the potency of the specific compound employed, the metabolic stability and length of action of that compound, the patient's age, body weight, general health, sex, diet, mode and time of administration, rate of excretion, drug combination, the severity of the condition to be treated, and the patient undergoing therapy. The daily dosage may, for example, range from about 0.001 mg to about 100 mg per kilo of body weight, administered singly or multiply in doses, e.g. from about 0.01 mg to about 25 mg each. Normally, such a dosage is given orally but parenteral administration may also be chosen.

### DEFINITIONS

"Optional" or "optionally" means that the subsequently described event or circumstance may, but need not, occur, and that the description includes instances where the event or circumstance occurs and instances in which it does not.

The term "heteroatom" means O, N, or S.

The term "(C₁-Cₙ)alkyl" denotes a straight, branched or cyclic or partially cyclic alkyl group having from 1 to n carbon atoms, i.e. 1, 2, 3... or n carbon atoms. For the "(C₁-Cₙ)alkyl" group to comprise a cyclic portion it should be formed of at least three carbon atoms. For parts of the range "(C₁-Cₙ)alkyl" all subgroups thereof are contemplated. For example, in the range (C₁-C₆)alkyl, all subgroups such as (C₁-C₅)alkyl, (C₁-C₄)alkyl, (C₁-C₃)alkyl, (C₁-C₂)alkyl, (C₁)alkyl, (C₂-C₆)alkyl, (C₂-C₅)alkyl, (C₂-C₄)alkyl, (C₂-C₃)alkyl, (C₂)alkyl, (C₃-C₆)alkyl, (C₃-C₅)alkyl, (C₃-C₄)alkyl, (C₃)alkyl, (C₄-C₆)alkyl, (C₄-C₅)alkyl, (C₄)alkyl, (C₅-C₆)alkyl, (C₆)alkyl. Examples of "C₁-C₆ alkyl" include methyl, ethyl, n-propyl, isopropyl, cyclopropyl, n-butyl, isobutyl, sec-butyl, t-butyl, cyclobutyl, cyclopropylmethyl, branched or cyclic or partially cyclic pentyl and hexyl Etc.

The term "halo-(C₁-Cₙ)alkyl" denotes a C₁-Cₙ alkyl as described above substituted with at least one halogen atom, which is preferably, F, Cl, Brand I, more preferably F and Cl, and most preferably F.

When a term denotes a range, for instance "1 to 6 carbon atoms" in the definition of (C₁-C₆)alkyl, each integer is considered to be disclosed, i.e. 1, 2, 3, 4, 5 and 6.

The term "(C₂-Cₙ)alkenyl" denotes a straight, branched or cyclic or partially cyclic alkyl group having at least one carbon-carbon double bond, and having from 2 to 6 carbon atoms. The alkenyl group may comprise a ring formed of 3 to 6 carbon atoms. For parts of the range "(C₂-Cₙ)alkenyl" all subgroups thereof are contemplated. For example, the range "(C₂-C₄)alkenyl" covers (C₂-C₄)alkenyl, (C₂-C₃)alkenyl, (C₂)alkenyl. Examples of "(C₂-C₄)alkenyl" include 2-propenyl, 2-butenyl, 3-butenyl, 2-methyl-2-propenyl Etc.

The term "(C₁-C₄)alkoxy" denotes -O-((C₁-C₄)alkyl) in which a (C₁-C₄)alkyl group is as defined above and is attached to the remainder of the compound through an oxygen atom. Examples of "(C₁-C₄)alkoxy" include methoxy, ethoxy, n-propoxy, isopropoxy, n-butoxy, isobutoxy, sec-butoxy and t-butoxy.

The term "halo(C₁-C₄)alkoxy" denotes a (C₁-C₄)alkoxy as described above substituted with a halogen atom, which is preferably, F, Cl, Br and I, more preferably F and Cl, and most preferably F.

The term "halo" means a halogen atom, and is preferably, F, Cl, Br and I, more preferably F and Cl, and most preferably F.

The term "3- to 10-membered heterocyclic ring" denotes a non-aromatic ring system having 3 to 10 ring atoms, in which at least one ring atoms is a heteroatom.

"An effective amount" refers to an amount of a compound of the invention that confers a therapeutic effect on the treated subject. The therapeutic effect may be objective (i.e. measurable by some test or marker) or subjective (i.e. subject gives an indication of or feels an effect).

As used herein, the terms "administration" or "administering" mean a route of administration for a compound disclosed herein. Exemplary routes of administration include, but are not limited to, oral, intraocular, intravenous, intraperitoneal, intraarterial, and intramuscular. The preferred route of administration can vary depending on various factors, e.g. the components of the pharmaceutical composition comprising a compound disclosed herein, site of the potential or actual disease and severity of disease.

The terms "subject" and "patient" are used herein interchangeably. They refer to a human or another mammal (e.g., mouse, rat, rabbit, dog, cat, cattle, swine, sheep, horse or primate) that can be afflicted with or is susceptible to a disease or disorder but may or may not have the disease or disorder. It is preferred that the subject is human.

Compounds of the invention may be disclosed by the name or chemical structure. If a discrepancy exists between the name of a compound and its associated chemical structure, then the chemical structure prevails.

The invention will now be further illustrated by the following non-limiting examples. The specific examples below are to be construed as merely illustrative, and not limitative of the remainder of the disclosure in any way whatsoever. Without further elaboration, it is believed that one skilled in the art can, based on the description herein, utilise the present invention to its fullest extent. All references and publications cited herein are hereby incorporated by reference in their entirety.

### Preparation of Compounds of the Invention

The compounds of the invention can be prepared according to the following General Synthetic Schemes by methods well known and appreciated in the art. Suitable reaction conditions, for the steps of these schemes, are well known in the art and appropriate substitutions of solvents and co-reagents are within the common general knowledge of the person skilled in the art. Likewise, it will be appreciated by those skilled in the art that synthetic intermediates may be isolated and/or purified by various well-known techniques as needed or desired, and that frequently, it will be possible to use various intermediates directly in subsequent synthetic steps with little or no purification. Furthermore, the skilled person will appreciate that in some circumstance, the orders in which moieties are introduced is not critical. The particular order of steps required to produce the compounds of formula (I) is dependent upon the particular compound being synthesized, the starting compound, and the relative liability of the substituted moieties as is well appreciated by those of ordinary skill in the art. All substituents, unless otherwise indicated, are as previously defined, and all reagents are well known and appreciated in the art.

The compounds of general formula (I) may be prepared by a variety of procedures, some of which are described below. One of the methods particularly suitable for the preparation of small to medium-sized peptides, as is well known and appreciated by those of ordinary skill in the art, is solid phase peptide synthesis (SPPS).

Suitable starting materials and protected amino acids of general formula AA-1, AA-2, Int-1, Int-2 or Int-3 are either commercially available or may be prepared by a variety of methods. For example, as illustrated in Scheme 1, the carboxylic acid functionality of appropriately substituted amino acids of general formula AA-1, can be chemoselectively protected as a suitable derivative, for example as a methyl ester, using well established procedures and reagents like a mixture of methanol and thionyl chloride to yield a compound of general formula Int-1. In a subsequent step, protection of the free amine functionality in Int-1 as, for example, an amide or a carbamate, like Fmoc, affords and intermediate of general structure Int-2. The intermediate of general formula Int-3 can be obtained by selective deprotection of the masked acid functionality present in Int-2, using for example an acidic hydrolysis. When using SPPS, Int-3 can be attached to a suitably functionalised resin, for example Wang resin, using ester formation reagents like, for exemplification, a mixture of diisopropylmethanediimine, 4-methylmorpholine and N,N-dimethylpyridin-4-amine in dichloromethane, to afford solid supported intermediate of general formula Int-4.

### GENERAL SYNTHETIC PROCEDURE 1

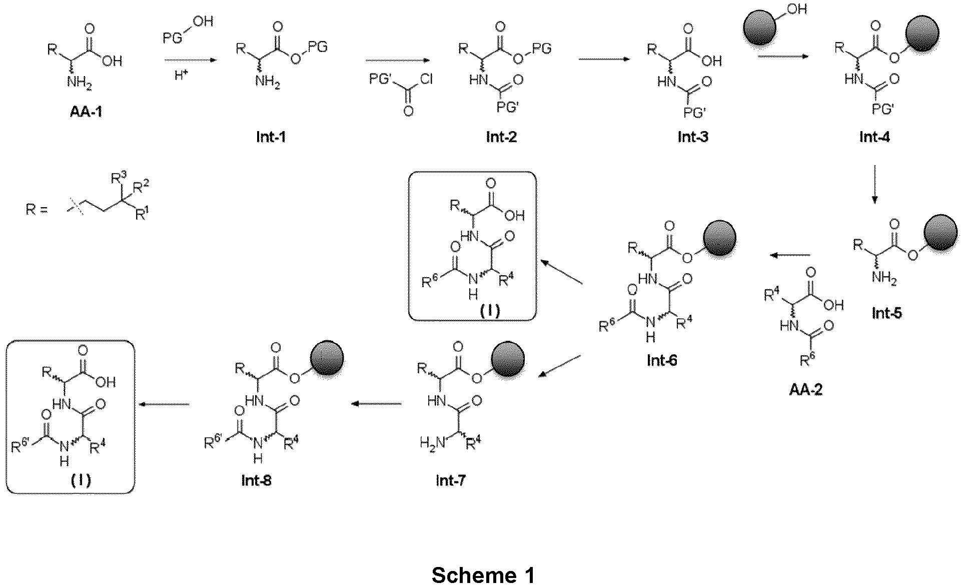

When required the resin bound intermediates Int-4 to Int-7 can be analysed after cleavage from resin. Alternatively, the corresponding steps on solid support can be performed without analysis. The protecting group of the amine in Int-4 can be removed by treatment with a base like piperidine, when, for example, the Fmoc protecting group is used, to yield Int-5. The free acid functionality present in the second N-protected-amino-acid, represented here by the compound of general formula AA-2, can be coupled with the free amine present in intermediate of general formula Int-5 to afford Int-6, using a classical amide coupling procedure, for example a mixture of HATU and a base, like N-methyl morpholine in a suitable solvent system, like a mixture of DCM-DMF. When, advantageously, the N-protecting group in AA-2 and Int-6 has the desired substitution, R⁶, cleavage, for example, by acidic hydrolysis using a reagent like trifluoroacetic acid, of the ester bond between the solid support and the compound of general structure represented in Int-6 yields the desired compound of general formula (I).

Alternatively, AA-2 can be introduced in Int-6 with a suitable protecting group on the amine moiety that can be removed in the subsequent step, to give Int-7, using a base like piperidine, when, for example, the Fmoc protecting group is used. The free basic amine functionality present in Int-7 can be readily functionalized as an acyl derivative using a classical amide coupling procedure, for example a mixture of HATU and a base, such as *N*-methyl morpholine in a suitable solvent system, like a mixture of DCM-DMF, to yield intermediate of general formula Int-8. The final compound of general formula (I) is obtained, by cleavage, for example, using acidic hydrolysis with a reagent like trifluoroacetic acid, of the ester bond between the resin solid support and the compound of general structure shown in Int-8.

Compounds of general formula (I) can be obtained, also, as shown in general Scheme 2 below. The skilled artisan will appreciate that the amine functionality in compounds of general formula Int-1 can be coupled with the free carboxylic acid functionality present in compounds of general formula AA-2, using, as described above and for the purpose of exemplification, a mixture of a coupling reagent and a base, such as HATU and N-methyl morpholine in a suitable solvent system, like DCM, DMF or a mixture thereof, to yield intermediate of general formula Int-9. The chemoselective cleavage of the ester-type protecting group present in compound of general formula Int-9 can then be done to afford the desired compounds of general formula (I), for example by basic hydrolysis in an aqueous media, such as LiOH in a mixture of water and acetonitrile.

### GENERAL SYNTHETIC PROCEDURE 2

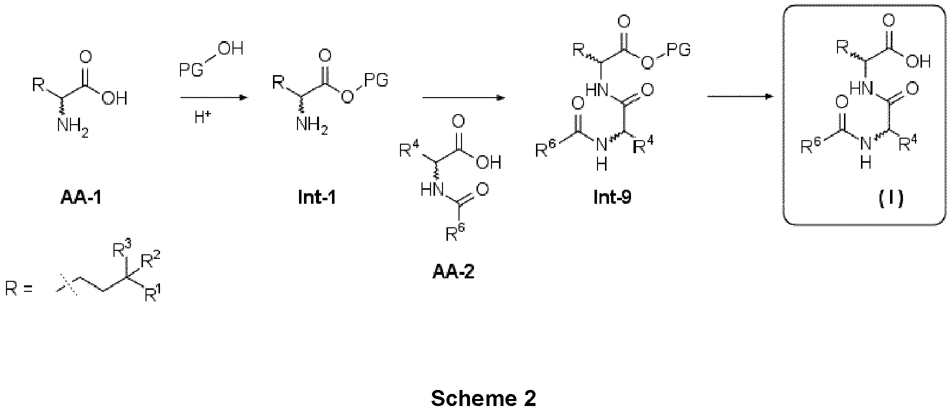

The products of each step can then be recovered by conventional methods including extraction, evaporation, precipitation, chromatography, filtration, trituration, crystallisation and the like.

Resin bound intermediates were analysed after cleavage from resin. All the analysis of intermediate are of free acids.

The skilled artisan will also appreciate that not all of the substituents in the compounds of formula (I) will tolerate certain reaction conditions employed to synthesise the compounds. These moieties may be introduced at a convenient point in the synthesis, or may be protected and then deprotected as necessary or desired, as is well known in the art. The skilled artisan will appreciate that the protecting groups may be removed at any convenient point in the synthesis of the compounds of the present invention. Methods for introducing or removing protecting groups used in this invention are well known in the art; see, for example, Greene and Wuts, Protective Groups in Organic Synthesis, 4th Ed., John Wiley and Sons, New York (2006)¹⁶.

### EXAMPLES

### Abbreviations

approx: approximately; aq: aqueous; br: broad; *ca.: circa;* CDI: 1,1'-Carbonyldiimidazole; d: doublet; DCM: dichloromethane; DIC: *N,N'-*Di*is*opropylcarbodiimide; dioxane: 1,4-dioxane; DIPEA: diisopropylethylamine; DMF: dimethylformamide; eq.: equivalent; Et₃N: triethylamine; EtOAc: ethyl acetate; EtOH: ethanol; Fmoc: fluorenylmethoxycarbonyl; Boc: *tert-*butoxycarbonyl; h: hours; min: minutes: HATU: 2-(3*H*-[1,2,3]triazolo[4,5-b]pyridin-3-yl)-1,1,3,3-tetramethyl isouronium hexafluorophosphate(V); HPLC: high performance liquid chromatography; IPA, isopropanol; LC: liquid chromatography; m: multiplet; M: molar, molecular ion; MeCN: acetonitrile; MeOH: methanol; MS: mass spectrometry; NMR: nuclear magnetic resonance; PDA: photodiode array; q: quartet; rt: room temperature (ca. 20 °C); R_{T}: retention time; s: singlet, solid; SPPS: solid phase peptide synthesis. t: triplet; TBAF: tetrabutylammonium fluoride; TBME: *tert*-butyl methyl ether; TFA: trifluoroacetic acid; THF: tetrahydrofuran; UPLC: ultra-performance liquid chromatography; UV: ultraviolet.

Other abbreviations are intended to convey their generally accepted meaning.

### General Experimental Conditions

All starting materials and solvents were obtained either from commercial sources or prepared according to the literature citation. Reaction mixtures were magnetically stirred and reactions performed at room temperature (ca. 20 °C) unless otherwise indicated.

Column chromatography was performed on an automated flash chromatography system, such as a CombiFlash Rf system, using pre-packed silica (40 µm) cartridges, unless otherwise indicated.

¹H NMR spectra were recorded, at 400 MHz or at 500 MHz, on a Bruker Avance AV-I-400 instrument, a Bruker Avance AV-II-400 instrument or a Bruker Avance III-500 HD spectrometer, equipped with a Bruker 5 mm SmartProbe^{™}. Chemical shifts are expressed in parts per million (ppm) using either the central peaks of the residual protic solvent as references or relative to tetramethylsilane, as internal standard. The spectra were recorded at 298 K unless otherwise indicated. The following abbreviations or their combinations are used for multiplicity of NMR signals: br = broad, d = doublet, m = multiplet, q = quartet, quint = quintet, s = singlet and t = triplet.

### Analytical Methods 1 to 3:

Analytical UPLC-MS experiments to determine retention times and associated mass ions were performed using a Waters ACQUITY UPLC^{®} H-Class system, equipped with ACQUITY PDA Detector and ACQUITY QDa Mass Detector, running one of the analytical methods described below.

Analytical LC-MS experiments to determine retention times and associated mass ions were performed using an Agilent 1200 series HPLC system coupled to an Agilent 1956, 6100 or 6120 series single quadrupole mass spectrometer running one of the analytical methods described below.

Preparative HPLC purifications were performed either using a Waters X-Select CSH C18, 5 µm, 19x50 mm column using a gradient of Acetonitrile and water, both modified with 0.1% v/v formic acid, or on a Waters X-Bridge BEH C18, 5 µm, 19x50 mm column using a gradient of Acetonitrile and 10 mM ammonium bicarbonate (aq). Fractions were collected following detection by UV at a single wavelength measured by a variable wavelength detector.

### Method 1: Acidic 3 min method

Column: Waters ACQUITY UPLC^{®} CSH C18, 1.7 µm, 2.1x30 mm at 40 °C
Detection: UV at 254 nm unless otherwise indicated, MS by electrospray ionisation
Eluent A: 0.1% v/v Formic acid in water, Eluent B: 0.1% v/v Formic acid in Acetonitrile

Gradient:

| **Time** | **%A** | **%B** | **Flow rate (ml/min)** |
|---|---|---|---|
| 0.00 | 95 | 5 | 0.77 |
| 0.11 | 95 | 5 | 0.77 |
| 2.15 | 5 | 95 | 0.77 |
| 2.56 | 5 | 95 | 0.77 |
| 2.83 | 95 | 5 | 0.77 |
| 3.00 | 95 | 5 | 0.77 |

### Method 2: Basic 3 min method

Column: Waters ACQUITY UPLC^{®} BEH C18, 1.7 µm, 2.1x30 mm at 40 °C
Eluent A: 10 mM ammonium bicarbonate (aq), Eluent B: Acetonitrile
   (other parameters the same as Method 1)

### Method 3: Basic 4 min method

Column: Waters X-Bridge BEH C18, 2.5 µm, 4.6x30 mm at 40 °C
Detection: UV at 254 nm unless otherwise indicated, MS by electrospray ionisation
Eluent A: 10 mM ammonium bicarbonate (aq), Eluent B: MeCN

| **Time** | **%A** | **%B** | **Flow rate (ml/min)** |
|---|---|---|---|
| 0.0 | 95.0 | 5.0 | 2.5 |
| 3.0 | 5.0 | 95.0 | 2.5 |
| 3.01 | 5.0 | 95.0 | 4.5 |
| 3.6 | 5.0 | 95.0 | 4.5 |
| 3.7 | 95.0 | 5.0 | 2.5 |
| 4.0 | 95.0 | 5.0 | 2.5 |

### Analytical Methods 4 to 8:

### Method 4: LCMS_SC_BASE

Apparatus: Agilent 1260 Bin. Pump: G1312B, degasser; autosampler, ColCom, DAD: Agilent G1315C, 210, 220 and 220-320 nm, PDA 210-320 nm, MSD: Agilent LC/MSD G6130B ESI, pos/neg 100-1000;
Column: Waters XSelectTM CSH C18, 30x2.1mm, 3.5µm, Temp: 25 °C, Flow: 1 mL/min, Gradient: t0 = 5% B, t1.6min = 98% B, t3min = 98% B, Post time: 1.4 min
Eluent A: 10mM ammonium bicarbonate in water (pH=9.0), Eluent B: acetonitrile

### Method 5: SC_ACID

Apparatus: Agilent 1260 Bin. Pump, degasser; autosampler, ColCom, DAD: Agilent G1315D, 210, 220 and 220-320 nm, PDA: 210-320 nm, MSD: Agilent LC/MSD G6130B ESI, pos/neg 100-1000, ELSD Alltech 3300 gas flow 1.5 ml/min, gas temp: 40 °C
Column: Waters XSelectTM C18, 30x2.1 mm, 3.5µm, Temp: 40 °C
Flow: 1 mL/min, Gradient: t0 = 5% B, t1.6min = 98% B, t3min = 98% B, Post time: 1.3 min
Eluent A: 0.1% formic acid in water, Eluent B: 0.1% formic acid in acetonitrile

### Method 6: UPLC_AN_BASE

Apparatus: Waters IClass; Bin. Pump: UPIBSM, SM: UPISMFTN with SO; UPCMA, PDA: UPPDATC, 210-320 nm, SQD: ACQ-SQD2 ESI; ELSD: gas pressure 40 psi, drift tube temp: 50 °C
Column: Waters XSelect CSH C18, 50x2.1mm, 2.5µm, Temp: 25 °C, Flow: 0.6 mL/min, Gradient: t0 = 5% B, t2.0min = 98% B, t2.7min = 98% B, Post time: 0.3 min
Eluent A: 10mM ammonium bicarbonate in water (pH=9.5), Eluent B: acetonitrile

### Method 7: UPLC_AN_ACID

Apparatus: Waters IClass; Bin. Pump: UPIBSM, SM: UPISMFTN with SO; UPCMA, PDA: UPPDATC, 210-320 nm, SQD: ACQ-SQD2 ESI; ELSD: gas pressure 40 psi, drift tube temp: 50 °C
Column: Waters XSelect CSH C18, 50x2.1mm, 2.5µm, Temp: 40 °C, Flow: 0.6 mL/min, Gradient: t0 = 5% B, t2.0min = 98% B, t2.7min = 98% B, Post time: 0.3 min
Eluent A: 0.1% formic acid in water, Eluent B: 0.1% formic acid in acetonitrile

### Method 8: PREP_ACID-AS4A

Apparatus: Agilent Technologies G6130B Quadrupole; HPLC instrument type: Agilent Technologies 1290 preparative LC; Column: Waters XSelect CSH (C18, 100x30mm, 10µ); Flow: 55 ml/min
Column temp: RT
Eluent A: 0.1% formic acid in water; Eluent B: 100% acetonitrile lin. gradient: t=0 min 20% B, t=2 min 20% B, t=8.5 min 60% B, t=10 min 100% B, t=13 min 100% B; Detection: DAD (220-320 nm); Detection: MSD (ESI pos/neg) mass range: 100 - 1000; fraction collection based on MS and DAD.

### Example 1

### (S)-2-((S)-2-acetamido-3-phenylpropanamido)-5,5-dimethylhexanoic acid.

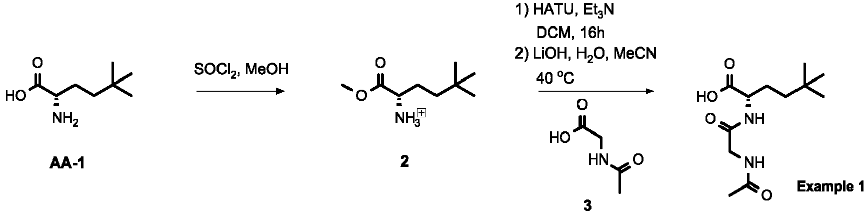

### Synthesis of methyl (S)-2-amino-5,5-dimethylhexanoate hydrochloride (2)

Thionyl chloride (2.75 mL, 37.7 mmol) was added dropwise to a mixture of 5,5-Dimethyl-L-norleucine **(AA-1,** 2.0 g, 12.6 mmol) in methanol (10 mL). The mixture was stirred at 50 °C for 1 hour. The solvent was evaporated *in vacuo* resulting in methyl (S)-2-amino-5,5-dimethylhexanoate hydrochloride **(2;** 2.59 g, 12.4 mmol, 98 % yield) as slight yellow solid. LCMS (Method 8, 0.987 min; M+H = 174.2; calculated 174.2)

### Synthesis of (S)-2-((S)-2-acetamido-3-phenylpropanamido)-5,5-dimethylhexanoic acid (Example 1)

HATU (110 mg, 0.289 mmol) was added to a mixture of acetyl glycine **3** (33.8 mg, 0.289 mmol) and triethylamine (0.121 ml, 0.866 mmol) in dichloromethane (1 ml). The mixture was stirred for 30 minutes. Intermediate Methyl (S)-2-amino-5,5-dimethylhexanoate hydrochloride **(2,** 50 mg, 0.238 mmol) was added and the mixture was stirred overnight. The mixture was concentrated *in vacuo.* The residue was dissolved in water (1 ml) and acetonitrile (1 ml). Lithium hydroxide (69.1 mg, 2.89 mmol) was added. The mixture was stirred overnight at 40°C. Concentrated *in vacuo* and purified by preparative HPLC (Method 8). The product containing fractions were combined and lyophilized resulting in the title compound, **Example 1** (27.6 mg, 0.107 mmol, 37 % yield, 98.5% purity). LCMS (Method 6, 0.953 min; M+H = 259.1; calculated 259.1). ¹H-NMR (400 MHz, DMSO) δ 12.68 (br, 1H), 8.12 - 8.01 (m, 2H), 4.14 (td, *J* = 8.1, 5.0 Hz, 1H), 3.73 - 3.69 (m, 2H), 1.84 (s, 3H), 1.66 (m, 1H), 1.56 (m, 1H), 1.26 - 1.10 (m, 2H), 0.85 (s, 9H).

### Example 2

### (2S)-5,5-dimethyl-2-{2-[(pyrazin-2yl)formamido]acetamido}hexanoic acid

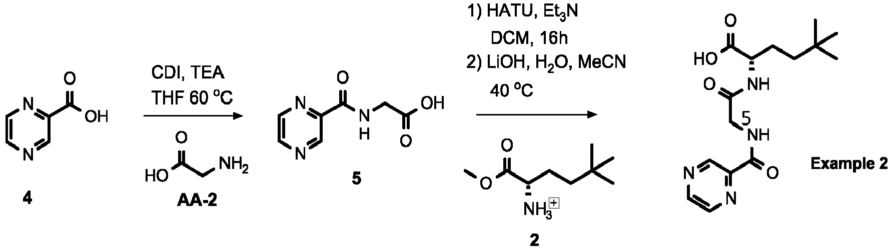

### Synthesis of (pyrazine-2-carbonyl) glycine (5).

CDI (324 mg, 2.000 mmol) was added to a suspension of pyrazine-2-carboxylic acid **(4,** 248 mg, 2 mmol) in tetrahydrofuran (dry) (3 ml). The mixture was heated at 60 °C for 1h, cooled to room temperature and triethylamine (300 µL, 2.152 mmol) and glycine **(AA-2,** 150 mg, 2 mmol) were added. The mixture was concentrated and water (10 mL) and EtOAc (25 mL) were added. The layers were separated and washed with brine (10 mL) the organic layer was dried over Na₂SO₄ and concentrated to afford a white solid (402 mg). The crude product was purified by column chromatography (12g SiO₂; 10% Methanol (7M NH3) in DCM) to afford (pyrazine-2-carbonyl) glycine **(5,** 82 mg, 0.453 mmol, 22 % yield). LCMS (Method 4, 0.162 min; M+H = 182.0; calculated 182.1)

### Synthesis of (2S)-5,5-dimethyl-2-{2-[(pyrazin-2-yl)formamido]acetamido}hexanoic acid (Example 2)

HATU (110 mg, 0.289 mmol) was added to a solution of (pyrazine-2-carbonyl) glycine **(5,** 52.3 mg, 0.289 mmol) and TEA (0.121 ml, 0.866 mmol) in dichloromethane (2 ml). The mixture was stirred for 30 minutes. Intermediate Methyl (S)-2-amino-5,5-dimethylhexanoate hydrochloride **(2** ,50 mg, 0.238 mmol) was added and the mixture was stirred overnight. The solvent was removed in vacuo and lithium hydroxide (69.1 mg, 2.89 mmol), acetonitrile (1 mL) and water (2 mL) were added. The mixture was stirred overnight at 40 °C. The mixture was concentrated *in vacuo* and purified by preparative HPLC, (Method 8). The fractions were combined and freeze-dried to afford the title compound (2S)-5,5-dimethyl-2-{2-[(pyrazin-2-yl)formamido]acetamido} hexanoic acid **(Example 2,** 12.4 mg, 0.038 mmol, 16 % yield, 99.1% purity). LCMS (Method 6, 0.729 min; M+H = 323.2; calculated 323.2). ¹H-NMR (400 MHz, DMSO) δ 9.19 (d, *J* = 1.6 Hz, 1H), 9.01 (t, *J* = 5.9 Hz, 1H), 8.90 (d, *J* = 2.5 Hz, 1H), 8.82 - 8.71 (m, 1H), 7.98 (d, *J* = 7.5 Hz, 1H), 7.68 (br, 1H), 4.03 (td, *J* = 7.4, 5.1 Hz, 1H), 3.96 (d, *J* = 5.9 Hz, 2H), 1.66 (m, 1H), 1.54 (m, 1H), 1.14 (dd, *J* = 9.8, 7.4 Hz, 2H), 0.82 (s, 9H).

### Example 3

### (2S)-2-[(2S)-2-acetamido-3-phenylpropanamido]-5,5-dimethylhexanoic acid.

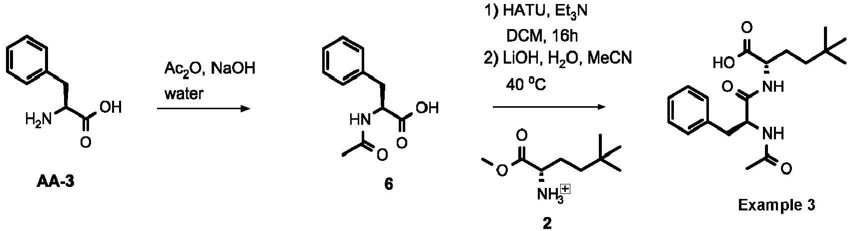

### Synthesis of acetyl-L-phenylalanine (6)

Aqueous sodium hydroxide (1M) (1.5 ml, 1.5 mmol) was added dropwise to a stirred solution of L-phenylalanine **(AA-2,** 248 mg, 1.5 mmol) in water (3 ml) until pH 12. Acetic anhydride (0.235 ml, 2.490 mmol) was added dropwise. More aqueous sodium hydroxide (1M) was added when a precipitation was formed to keep pH > 8. The mixture was stirred for 3hours. The pH was adjusted to pH 2 with 5M aqueous hydrochloric acid, the precipitated product was collected by filtration and dried *in vacuo* to afford acetyl-L-phenylalanine **(6,** 274 mg, 1.322 mmol, 88 % yield). LCMS (Method 5, 1.465 min; M+H = 208.1; calculated 208.1).

### Synthesis of (S)-2-((S)-2-acetamido-3-phenylpropanamido)-5,5-dimethylhexanoic acid (Example 3)

HATU (110 mg, 0.289 mmol) was added to a mixture of acetyl-L-phenylalanine **(6,** 59.8 mg, 0.289 mmol) and triethylamine (0.121 ml, 0.866 mmol) in dichloromethane (1 ml). The mixture was stirred for 30 minutes. Intermediate methyl (S)-2-amino-5,5-dimethylhexanoate hydrochloride **(2,** 50 mg, 0.238 mmol) was added and the mixture was stirred overnight. The solvent was removed in vacuo and lithium hydroxide (69.1 mg, 2.89 mmol), acetonitrile (1 ml) and water (1 ml) were added. The mixture was stirred overnight at 40°C. The mixture was concentrated *in vacuo* and purified by preparative HPLC, (Method 8). The fractions were combined and freeze-dried to afford the title compound (S)-2-((S)-2-acetamido-3-phenylpropanamido)-5,5-dimethylhexanoic acid **(Example 3,** 20.7 mg, 0.059 mmol, 24 % yield, 95.75% purity). LCMS (Method 7, 1.274 min; M+H = 349.2; calculated 349.2). ¹H-NMR (400 MHz, DMSO) δ 12.58 (s, 1H), 8.20 (d, *J* = 7.8 Hz, 1H), 8.08 (d, *J* = 8.6 Hz, 1H), 7.27 (s, 2H), 7.26 - 7.22 (m, 2H), 7.18 (m, 1H), 4.56 (ddd, *J* = 10.2, 8.4, 3.9 Hz, 1H), 4.14 (td, *J* = 8.1, 5.0 Hz, 1H), 3.00 (dd, *J* = 13.9, 3.9 Hz, 1H), 2.70 (dd, *J* = 13.9, 10.2 Hz, 1H), 1.73 (s, 3H), 1.72 - 1.66 (m, 1H), 1.66 - 1.51 (m, 1H), 1.30 - 1.08 (m, 2H), 0.86 (s, 9H).

### Example 4

### (2S)-2-[(2S)-2-acetamido-3-(4-fluorophenyl)propanamido]-5,5-dimethylhexanoic acid

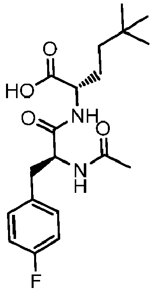

Example 4 was prepared in an analogous manner to Example 3, starting from its corresponding amino acid analogue to AA-3.

(2S)-2-[(2S)-2-acetamido-3-(4-fluorophenyl)propanamido]-5,5-dimethylhexanoic acid **(Example 4,** 28.6mg, 27% yield, 100% purity) LCMS (Method 7, 1.308 min, M+H = 367.2, calculated 367.2). ¹H-NMR (400 MHz, DMSO) δ 12.57 (s, 1H), 8.21 (d, J = 7.7 Hz, 1H), 8.08 (d, J = 8.7 Hz, 1H), 7.29 (dd, J = 8.6, 5.7 Hz, 2H), 7.08 (t, J = 8.9 Hz, 2H), 4.55 (ddd, J = 10.1, 8.5, 4.1 Hz, 1H), 4.14 (td, J = 8.1, 5.0 Hz, 1H), 2.97 (dd, J = 13.9, 4.2 Hz, 1H), 2.68 (dd, J = 13.9, 10.1 Hz, 1H), 1.74 (s, 3H), 1.72 - 1.64 (m, 1H), 1.64 - 1.49 (m, 1H), 1.28 - 1.11 (m, 2H), 0.86 (s, 9H).

### Example 5

### (2S)-2-[(2S)-3-(3,5-difluorophenyl)-2-acetamidopropanamido]-5,5-dimethylhexanoic acid

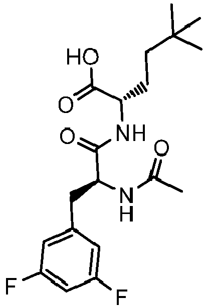

Example 5 was prepared in an analogous manner to Example 3, starting from its corresponding amino acid analogue to AA-3.

(2S)-2-[(2S)-3-(3,5-difluorophenyl)-2-acetamidopropanamido]-5,5-dimethylhexanoic acid **(Example 5,** 27.8 mg, 30% yield, 98.13% purity) LCMS (Method 7, 1.308 min; M+H = 367.2; calculated 367.2). ¹H-NMR (400 MHz, DMSO) δ 12.64 (s, 1H), 8.22 (d, J = 7.8 Hz, 1H), 8.10 (d, J = 8.6 Hz, 1H), 7.05 (tt, J = 9.5, 2.4 Hz, 1H), 7.01 -6.97 (m, 2H), 4.60 (td, J = 9.8, 4.2 Hz, 1H), 4.14 (td, J = 8.1, 4.9 Hz, 1H), 3.00 (dd, J = 13.9, 4.2 Hz, 1H), 2.74 (dd, J = 13.7, 10.0 Hz, 1H), 1.75 (s, 3H), 1.74 - 1.64 (m, 1H), 1.63 - 1.47 (m, 1H), 1.26 - 1.12 (m, 2H), 0.86 (s, 9H).

### Example 6

### (2S)-2-[(2S)-2-acetamido-3-(1H-indol-3-yl)propanamido]-5,5-dimethylhexanoic acid.

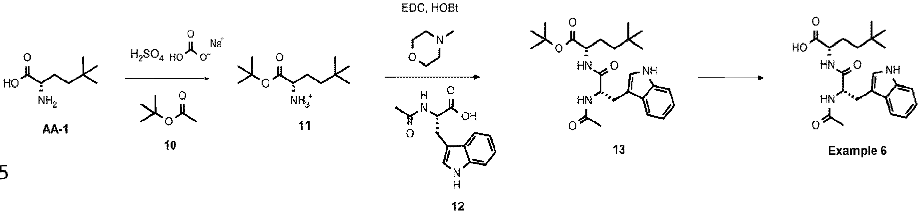

### Synthesis of tert-butyl (S)-2-amino-5,5-dimethylhexanoate (11)

H₂SO₄ (0.62 g, 0.33 mL, 2.0 Eq, 6.3 mmol) was added to a stirred suspension of (S)-2-amino-5,5-dimethylhexanoic acid (**AA-1,** 0.50 g, 1 Eq, 3.1 mmol) and 4A molecular sieves in *tert*-butyl acetate **(10,** 8.7 g, 10 mL, 24 Eq, 75 mmol) whilst maintaining the temperature below 5 °C. The reaction was then allowed to attain room temperature and stirred for 20 hours.

The reaction was added dropwise to a vigorously stirred solution of sodium bicarbonate (2.6 g, 10.0 Eq, 31 mmol) in water (20 mL) and the resulting mixture extracted with EtOAc (2 x 20 mL), dried (MgSO₄), filtered and evaporated in vacuo to afford a light brown gum. The gum was azeotroped with toluene to afford the title compound (2S)-2-[(2S)-2-acetamido-3-(1H-indol-3-yl)propanamido]-5,5-dimethylhexanoic acid (**11**, 0.54 g, 2.4 mmol, 76 %, 95%) as a cream solid. UPLC (CSH C18 Column, 130Å, 1.7 µm, 2.1 mm x 30 mm, 3 min method, 0.1% Formic acid, 2-100 % MeCN/water): m/z 216.4 (M+H)⁺ (ES+); at 0.86 min. ¹H NMR (500 MHz, DMSO-d6) δ 7.50 (s, 2H), 3.71 (app. t, J = 5.8 Hz, 1H), 1.71 - 1.62 (m, 2H), 1.45 (s, 9H), 1.33 - 1.24 (m, 1H), 1.16 - 1.06 (m, 1H), 0.86 (s, 9H).

### Synthesis of tert-butyl (S)-2-((S)-2-acetamido-3-(1H-indol-3-yl)propanamido)-5,5-dimethylhexanoate (13)

HOBt (0.34 g, 1.1 Eq, 2.2 mmol) was added to a stirred solution of acetyl-L-tryptophan **(12,** 0.50 g, 1 Eq, 2.0 mmol) and *tert*-butyl (*S*)-2-amino-5,5-dimethylhexanoate **(11,** 0.46 g, 1.05 Eq, 2.1 mmol) in DMF (5 mL). The reaction was cooled to 0 °C, EDC (0.41 g, 1.05 Eq, 2.1 mmol) and 4-methylmorpholine (0.41 g, 0.45 mL, 2.0 Eq, 4.1 mmol) were added, allowed to attain room temperature and stirred for 16 hours.

The reaction was quenched with water (20 mL), extracted with EtOAc (2 x 20 mL), washed with water (2 x 10 mL) and brine (5 mL), dried (MgSO₄), filtered, evaporated in vacuo and purified by chromatography on silica gel (40 g FlashPure column, 0 - 100% EtOAc/isohexane to afford the title compound *tert*-butyl (S)-2-((*S*)-2-acetamido-3-(1H-indol-3-yl)propanamido)-5,5-dimethylhexanoate **(13,** 0.56 g, 1.2 mmol, 62 %, 99% Purity), 3318-07-1, as a colourless foam. LCMS (XBridge BEH C18, 130Å, 2.5 µm, 2.1 mm x 30 mm, 3 min method, 0.1% Ammonium Hydroxide, 5-100% MeCN/water): 3318-07-1, m/z 444.0 (M+H)⁺ (ES+); at 2.18 min, 99.5% purity at 210-400nm. ¹H NMR (500 MHz, DMSO-*d*₆) δ 10.79 (d, *J* = 2.4 Hz, 1H), 8.23 (d, *J* = 7.4 Hz, 1H), 8.02 (d, *J* = 8.4 Hz, 1H), 7.62 (d, *J* = 7.9 Hz, 1H), 7.34 - 7.29 (m, 1H), 7.13 (d, *J* = 2.0 Hz, 1H), 7.05 (ddd, J *=* 8.1, 7.0, 1.2 Hz, 1H), 6.97 (ddd, *J* = 8.0, 6.9, 1.1 Hz, 1H), 4.64 - 4.56 (m, 1H), 4.11 -4.03 (m, 1H), 3.10 (dd, *J* = 14.7, 4.3 Hz, 1H), 2.87 (dd, *J* = 14.7, 9.7 Hz, 1H), 1.75 (s, 3H), 1.70 - 1.52 (m, 2H), 1.40 (s, 9H), 1.21 - 1.14 (m, 2H), 0.86 (s, 9H).

### Synthesis of (2S)-2-[(2S)-2-acetamido-3-(1H-indol-3-yl)propanamido]-5,5-dimethylhexanoic acid (Example 6)

*Tert*-butyl (*S*)-2-((*S*)-2-acetamido-3-(1H-indol-3-yl)propanamido)-5,5-dimethylhexanoate **13** (480 mg, 1 Eq, 1.08 mmol) was dissolved in ice/cold 2,2,2-trifluoroacetic acid (50 mL), the ice bath removed and stirred for 45 min. The TFA was evaporated in vacuo at 25 °C, azeotroped with toluene (50 mL) and then dissolved in DCM (10 mL) and adsorbed onto celite. The DCM was evaporated *in vacuo* and the material purified by chromatography on RP Flash C18 (24 g cartridge, 5-50% MeCN/10 mM Ammonium Bicarbonate) to afford the title compound (2S)-2-[(2S)-2-acetamido-3-(1H-indol-3-yl)propanamido]-5,5-dimethylhexanoic acid **(Example 6,** 218 mg, 0.56 mmol, 51 %, 99%) as a white solid. UPLC (CSH C18 Column, 130Å, 1.7 µm, 2.1 mm x 30 mm, 3 min method, 0.1% Formic acid, 2-100% MeCN/water) m/z 388.9 (M+H)+ (ES+); at 1.27 min, 100% purity 210-400nm. LCMS (XBridge BEH C18, 130Å, 2.5 µm, 2.1 mm x 30 mm, 3 min method, 0.1% Ammonium Hydroxide, 5-100% MeCN/water): 3318-14-1, m/z 388.0 (M+H)⁺ (ES+); at 1.05 min, 99% purity at 260nm +/- 80nm. ¹H NMR (500 MHz, DMSO-*d*₆) δ 10.78 (s, 1H), 8.10 (d, *J =* 8.3 Hz, 1H), 7.86 (d, *J=* 7.2 Hz, 1H), 7.58 (d, *J* = 7.9 Hz, 1H), 7.31 (dt, *J* = 8.1, 1.0 Hz, 1H), 7.12 (d, *J* = 2.2 Hz, 1H), 7.05 (ddd, *J* = 8.1, 6.9, 1.2 Hz, 1H), 6.97 (ddd, *J* = 8.0, 7.0, 1.1 Hz, 1H), 4.54 - 4.46 (m, 1H), 4.01 - 3.93 (m, 1H), 3.12 (dd, *J* = 14.8, 4.3 Hz, 1H), 2.86 (dd, *J =* 14.8, 9.6 Hz, 1H), 1.76 (s, 3H), 1.74 - 1.65 (m, 1H), 1.61 - 1.50 (m, 1H), 1.19 - 1.11 (m, 2H), 0.83 (s, 9H), CO₂H proton not observed.

### Example 7

### (S)-5,5-dimethyl-2-((S)-3-phenyl-2-(pyrazine-2-carboxamido)propanamido)hexanoic acid.

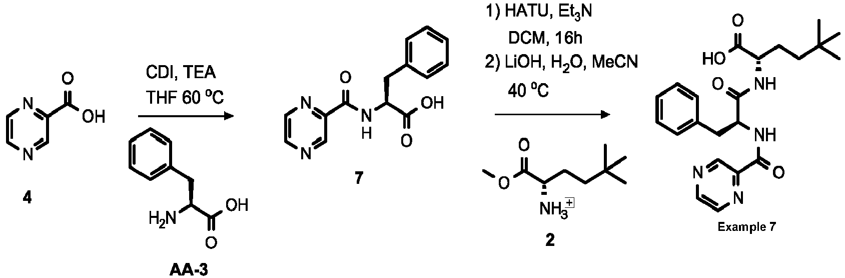

### Synthesis of (pyrazine-2-carbonyl)-L-phenylalanine (7)

CDI (324 mg, 2.000 mmol) was added to a suspension of pyrazine-2-carboxylic acid **(4,** 248 mg, 2 mmol) in Tetrahydrofuran (dry) (3 ml). The mixture was heated at 60 °C for 1h, cooled to room temperature and TEA (300 µL, 2.152 mmol) and L-phenylalanine (330 mg, 2 mmol) were added and the mixture was stirred for 2 hours. The solvent was evaporated and water and ethyl acetate were added, separated and extracted twice more with ethyl acetate. The organic layer was dried over Na₂SO₄ and concentrated *in vacuo* to afford (pyrazine-2-carbonyl)-L-phenylalanine **(7,** 80 mg, 0.295 mmol, 14.75 % yield). LCMS (Method 4, 1.379 min; (M+H)⁺= 272.0; calculated 272.1)

### Synthesis of (S)-5,5-dimethyl-2-((S)-3-phenyl-2-(pyrazine-2-carboxamido)propanamido)hexanoic acid (Example 7)

HATU (224 mg, 0.59 mmol) was added to a mixture of intermediate (S)-2-amino-5,5-dimethylhexanoic acid **(2,** 94 mg, 0.59 mmol), intermediate **(7,** pyrazine-2-carbonyl)-L-phenylalanine (160 mg, 0.590 mmol) and diisopropylethylamine (0.206 mL, 1.18 mmol) in *N,N*-Dimethylformamide (1 mL). The mixture was stirred at room temperature for 16 hours. The reaction mixture was purified by preparative HPLC, (Method 8). The product containing fractions were combined and lyophilised resulting in the title compound (S)-5,5-dimethyl-2-((S)-3-phenyl-2-(pyrazine-2-carboxamido)propanamido)hexanoic acid **(Example 7,** 14 mg, 0.034 mmol, 5.75 % yield). LCMS (Method 6, 0.958 min; M+H = 413.2; calculated 413.2). ¹H-NMR (400 MHz, DMSO) δ 12.77 (br, 1H), 9.12 (d, *J =* 1.5 Hz, 1H), 8.88 (d, *J* = 2.5 Hz, 1H), 8.81 - 8.71 (m, 1H), 8.69 (d, *J* = 8.6 Hz, 1H), 8.44 (d, *J* = 7.8 Hz, 1H), 7.31 - 7.10 (m, 5H), 4.90 - 4.79 (m, 1H), 4.26 - 4.10 (m, 1H), 3.17 (dd, *J* = 13.9, 4.6 Hz, 1H), 3.08 (dd, *J* = 13.8, 8.5 Hz, 1H), 1.80 - 1.66 (m, 1H), 1.66 - 1.56 (m, 1H), 1.30 - 1.12 (m, 2H), 0.84 (s, 9H).

### Example 8

### (2S)-5,5,5-trifluoro-2-[(2S,3S)-3-methyl-2-[(pyrazin-2-yl)formamido]pentanamido] pentanoic acid.

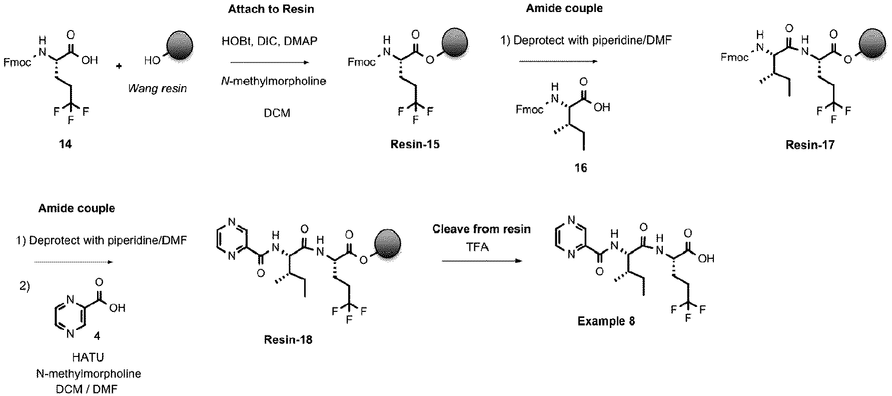

### Synthesis of (S)-2-((((9H-Fluoren-9-yl) methoxy) carbonyl) amino)-5,5,5-trifluoropentanoic acid (14)

(S)-2-((((9H-Fluoren-9-yl) methoxy) carbonyl) amino)-5,5,5-trifluoropentanoic acid **14,** was prepared by following the procedure shown in PCT Patent Publication WO 2010/132601 A1, to prepare methyl 5,5,5-trifluoropentanoate, followed by Fmoc protection and methyl ester hydrolysis, as described in WO 2015/131100 A1.

### Synthesis of (S)-2-((((9H-Fluoren-9-yl)methoxy)carbonyl)amino)-5,5,5-trifluoropentanoic acid [Resin bound on C-terminal] (Resin-15)

Wang Resin (Loading: 1.0 mmol/g, 90.0 mg, 0.09 mmol) was swollen in DCM (1 mL) for 10 min and filtered, washing with DCM (3 x 5 mL). (*S*)-2-((((9*H*-fluoren-9-yl)methoxy)carbonyl)amino)-5,5,5-trifluoropentanoic acid **(14,** 177 mg, 0.450 mmol), 1*H*-benzo[*d*][1,2,3]triazol-1-ol hydrate (69.0 mg, 0.45 mmol), di*is*opropylmethanediimine (70.0 µL, 0.45 mmol), 4-methylmorpholine (49.0 µL, 0.45 mmol) and *N,N*-dimethylpyridin-4-amine (11.0 mg, 0.09 mmol) and DCM (5 mL) were added and the mixture was stirred at rt for 18 hours. The reaction mixture was filtered and washed with DMF (5 mL x 3), DCM (5 mL x 3), water (5 mL x 2) and DMF (5 mL x 3). DCM (5 mL), (S)-2-((((9H-fluoren-9-yl)methoxy)carbonyl)amino)-5,5,5-trifluoropentanoic acid **(14,** 177 mg, 0.45 mmol), 1*H*-benzo[*d*][1,2,3]triazol-1-ol hydrate (69.0 mg, 0.45 mmol), 4-methylmorpholine (49.0 µL, 0.45 mmol), di*is*opropylmethanediimine (69.7 µL, 0.45 mmol) and *N,N*-dimethylpyridin-4-amine (11.0 mg, 0.09 mmol) were added to the resin and the suspension was gently stirred for a further 12 hours at room temperature. The mixture was filtered and washed with DMF (5 mL x 3), DCM (5 mL x 3), water (5 mL x 2) and DMF (5 mL x 3) to afford the resin bound intermediate **Resin-15.** No analysis was undertaken at this stage and resin was used directly in the next step.

### Synthesis of (S)-2-((2S,3S)-2-((((9H-Fluoren-9-yl)methoxy)carbonyl)amino)-3-methylpentanamido)-5,5,5-trifluoropentanoic acid [Resin bound on C-terminal] (Resin-17)

(*S*)-2-((((9*H*-Fluoren-9-yl)methoxy)carbonyl)amino)-5,5,5-trifluoropentanoic acid, resin bound **(Resin-15,** 90.0 mg, 0.09 mmol) was swollen in DMF (1 mL) for 10 minutes and filtered. 20% piperidine in DMF (5 mL) was added to the swollen resin and the suspension was agitated with nitrogen gas at room temperature for 20 minutes. The suspension was filtered, 20% piperidine in DMF (5 mL) was added and the suspension was agitated with nitrogen gas at room temperature for 20 minutes. The suspension was filtered and washed with DMF (5 mL x 3), DCM (5 mL x 3), water (5 mL x 2) and DMF (5 mL x 3). The obtained solid was used without further purification.

A mixture of DMF/DCM (1:1, 3 mL) was added followed by (((9*H*-fluoren-9-yl)methoxy)carbonyl)-*L*-isoleucine (**16**, 127 mg, 0.36 mmol), HATU (137 mg, 0.36 mmol) and 4-methylmorpholine (40.0 µL, 0.36 mmol). The suspension was agitated with nitrogen gas at room temperature for 2 hours then filtered and washed with DMF (5 mL x 3), DCM (5 mL x 3), water (5 mL x 2) and DMF (5 mL x 3). DMF (3 mL) was added followed by (((9*H*-fluoren-9-yl)methoxy)carbonyl)-*L-*isoleucine **(16,** 127 mg, 0.36 mmol), HATU (137 mg, 0.36 mmol) and 4-methylmorpholine (40.0 µL, 0.36 mmol). The suspension was agitated with nitrogen gas at room temperature for 15 hours then filtered and washed with DMF (5 mL x 3), DCM (5 mL x 3), water (5 mL x 2) and DMF (5 mL x 3) to afford the resin bound intermediate **Resin-17.** A small portion of resin was added to 0.5 mL of TFA and the suspension was left at room temperature for 1 hour. The suspension was concentrated *in vacuo* to afford (*S*)-2-((2*S*,3*S*)-2-((((9*H*-fluoren-9-yl)methoxy)carbonyl)amino)-3-methylpentanamido)-5,5,5-trifluoropentanoic acid (77% purity, Method 1, 1.67 min; M+H = 507.4).

### Synthesis of (S)-5,5,5-trifluoro-2-[(2S,3S)-3-methyl-2-[(pyrazin-2-yl)formamido]pentanamido]pentanoic acid (Resin-18)

(*S*)-2-((2*S*,3*S*)-2-((((9H-fluoren-9-yl)methoxy)carbonyl)amino)-3-methylpentanamido)-5,5,5-trifluoropentanoic acid, resin bound **(Resin-17,** 30.0 mg, 0.030 mmol) was swollen in DMF at room temperature for 10 minutes and filtered. 20% Piperidine in DMF (5 mL) was added to the swollen resin, and nitrogen gas was passed through the suspension for 20 minutes. The suspension was filtered and 20% piperidine in DMF (5 mL) was added, and nitrogen gas was passed through the suspension for 20 minutes. The suspension was filtered and washed with DMF (5 mL x 3), DCM (5 mL x 3), water and DMF (5 mL x 3). The obtained solid was used without further purification. DMF (5 mL) was added to the obtained resin and pyrazine-2-carboxylic acid 4 (19 mg, 0.15 mmol), HATU (57 mg, 0.15 mmol) and 4-methylmorpholine (15 mg, 0.15 mmol) were added. Nitrogen gas was passed through the suspension for 2 hours. The suspension was filtered and washed with DMF (5 mL x 3), DCM (5 mL x 3), water (5 mL x 2) and DMF (5 mL x 3), and DMF (10 mL) was added. pyrazine-2-carboxylic acid **(4,** 19 mg, 0.15 mmol), HATU (57 mg, 0.15 mmol) and 4-methylmorpholine (15 mg, 0.15 mmol) were added. Nitrogen gas was passed through for 15 hours. The reaction suspension was filtered and washed with DMF (5 mL x 3), DCM (5 mL x 3), water (5 mL x 2), DMF (5 mL x 2) and DCM (5 mL x 2), to resin bound intermediate **Resin-18** (15 mg, 0.030 mmol).

A mixture of TFA/water (95:5, 1 mL) was added to the resin bound intermediate **Resin-18** (15 mg, 0.030 mmol) and stirred at room temperature for 3 hours. The reaction mixture was filtered, and resin washed with DCM (20 mL x 3). The combined filtrates were concentrated *in vacuo* and azeotroped with toluene (15 mL x 3). The crude product was purified by preparative HPLC. This resulted in the title compound, (S)-5,5,5-trifluoro-2-((2S,3S)-3-methyl-2-(pyrazine-2-carboxamido) pentanamido)pentanoic acid **(Example 8,** 6.3 mg, 16 µmol, 54% yield) as a white solid. 100% LCMS purity (Method 2, 0.56 min; M-H = 389.2), 100% NMR purity [¹H NMR (500 MHz, DMSO-d6) δ 12.84 (br s, 1H), 9.20 (s, 1H), 8.91 (d, J = 2.5 Hz, 1H), 8.77 (t, J = 2.1 Hz, 1H), 8.56 (d, J = 7.7 Hz, 1H), 8.48 (d, J = 9.0 Hz, 1H), 4.49 (dd, J = 9.1, 6.9 Hz, 1H), 4.35 - 4.26 (m, 1H), 2.39 - 2.21 (m, 2H), 2.01 - 1.77 (m, 3H), 1.57 - 1.45 (m, 1H), 1.17-1.06 (m, 1H), 0.93 (d, J = 6.8 Hz, 3H), 0.85 (t, J = 7.4 Hz, 3H)].

### Example 9

### (S)-5,5-dimethyl-2-[(2S,3S)-3-methyl-2-[(pyrazin-2-yl)formamido]pentanamido]hexanoic acid.

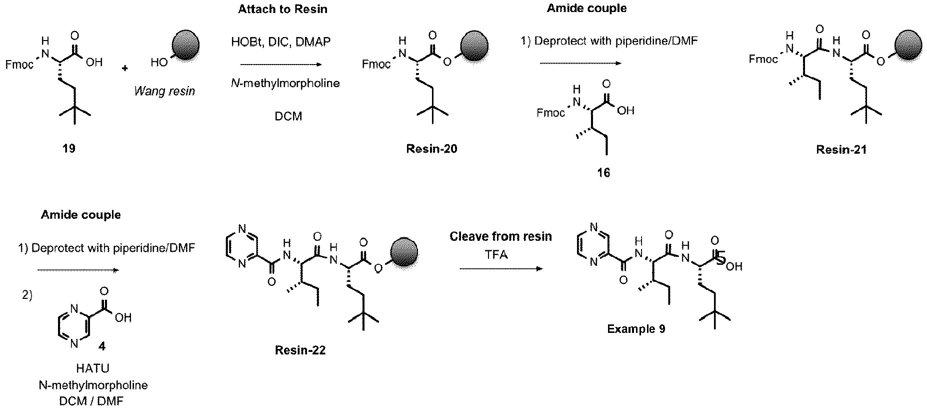

### Synthesis of (S)-2-((((9H-Fluoren-9-yl)methoxy)carbonyl)amino)-5,5-dimethylhexanoic acid [Resin bound on C-terminal] (Resin-20)

Wang resin (Loading: 1.0 mmol/g, 650 mg, 0.65 mmol) was swollen in DCM (30 mL) at room temperature for 10 minutes and filtered. DCM (30 mL) was added followed by (*S*)-2-((((9*H*-fluoren-9-yl)methoxy)carbonyl)amino)-5,5-dimethylhexanoic acid **(19,** 0.25 g, 0.65 mmol), DIC (0.31 mL, 2.00 mmol), *1H-*benzo[*d*][1,2,3]triazol-1-ol hydrate (0.30 g, 2.0 mmol), 4-methylmorpholine (0.21 mL, 2.00 mmol) and DMAP (79.0 mg, 0.65 mmol) and the suspension was gently stirred at room temperature for 16 hours. The suspension was filtered and washed with DMF (20 mL x 3), DCM (20 mL x 3), water (20 mL x 2), DMF (20 mL x 2) and DCM (20 mL x 2). A mixture of DCM/DMF (1:1, 30 mL) was added followed by (*S*)-2-((((9*H*-fluoren-9-yl)methoxy)carbonyl)amino)-5,5-dimethylhexanoic acid (**19**, 0.25 g, 0.65 mmol), DIC (0.31 mL, 2.00 mmol), 1*H*-benzo[*d*][1,2,3]triazol-1-ol hydrate (0.30 g, 2.00 mmol), 4-methylmorpholine (0.21 mL, 2.00 mmol) and DMAP (79.0 mg, 0.65 mmol). The suspension was stirred at room temperature for 24 hours then filtered and washed with DMF (5 mL x 2), DCM (5 mL x 2), water (5 mL x 2), DMF (5 mL x 2), DMF (20 mL x 2) and DCM (20 mL x 2) to afford the resin bound **Resin-20.** A small portion of the resin was added to TFA (0.05 mL) and the suspension was left at room temperature for 1 hour. The suspension was filtered, and concentrated *in vacuo* to afford (*S*)-2-((((9*H-*fluoren-9-yl)methoxy)carbonyl)amino)-5,5-dimethylhexanoic acid (100% purity), which was analysed by LCMS (Method 3, 1.53 min; M+Na = 404.1).

### Synthesis of (S)-2-((2S,3S)-2-((((9H-Fluoren-9-yl)methoxy)carbonyl)amino)-3-methylpentanamido)-5,5- dimethylhexanoic acid [Resin bound on C-terminal] (Resin-21)

(*S*)-2-((((9*H*-Fluoren-9-yl)methoxy)carbonyl)amino)-5,5-dimethylhexanoic acid, resin bound **(Resin-20,** 600 mg, 600 µmol) was swollen in DMF (1 mL) for 10 min then filtered. 20% Piperidine in DMF (30 mL) was added to the swollen resin and the suspension was agitated with nitrogen gas at room temperature for 20 minutes. The suspension was filtered and washed with DMF (20 mL x 2), DCM (20 mL x 2), water (20 mL x 2), DCM (20 mL x 2) and DMF (20 mL x 3). The suspension was filtered and 20% piperidine in DMF (30 mL) was added and nitrogen gas was passed through the suspension for 20 minutes. The suspension was filtered and washed with DMF (20 mL x 3), DCM (20 mL x 3), water (20 mL x 2) and DMF (20 mL x 3). The obtained solid was used without further purification.

DCM (2.6 mL) and DMF (0.4 mL) were added followed by additions of (((9H-fluoren-9-yl)methoxy)carbonyl)-*L*-isoleucine **(16,** 850 mg, 2.40 mmol), HATU (910 mg, 2.40 mmol) and 4-methylmorpholine (0.26 mL, 2.40 mmol). The reaction was stirred gently at room temperature for 16 hours under an atmosphere of nitrogen then filtered and washed with DMF (20 mL x 2), DCM (20 mL x 2), water (20 mL x 2), DCM (20 mL x 2) and DMF (20 mL x 3). (((9H-fluoren-9-yl)methoxy)carbonyl)-*L*-isoleucine **(16,** 850 mg, 2.40 mmol), HATU (910 mg, 2.40 mmol) and 4-methylmorpholine (0.26 mL, 2.40 mmol) were added. Nitrogen gas was passed through for 15 hours. The reaction suspension was filtered and the washed with DMF (20 mL x 3), DCM (20 mL x 3), water (20 mL x 2), DMF (20 mL x 2) and DCM (20 mL x 2), to afford resin bound intermediate **Resin-21.** A small portion of the resin was added to TFA (0.05 mL) and stirred at room temperature for 30 minutes. The suspension was filtered, washing with DCM (5 mL) and the combined filtrates were concentrated *in vacuo* to afford (*S*)-2-((2*S*,3*S*)-2-((((9*H-*Fluoren-9-yl)methoxy)carbonyl)amino)-3-methylpentanamido)-5,5-dimethylhexanoic acid (96% purity) which was analysed by LCMS (Method 2, 1.17 min; M+H = 496.56).

**Table 2: Intermediates Resin-23 and Resin-24 were prepared from the corresponding starting material by a manner analogous to Intermediate Resin-21.**

| **Intermediate** | **Structure** |
|---|---|
| **Resin-23** | 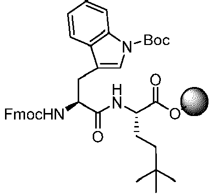 |
| **Resin-24** | 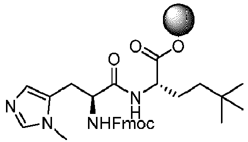 |

### Synthesis of (S)-5,5-Dimethyl-2-((2S,3S)-3-methyl-2-(pyrazine-2-carboxamido)pentanamido)hexanoic acid (Example 9)

(*S*)-2-((2*S*,3*S*)-2-((((9*H*-Fluoren-9-yl)methoxy)carbonyl)amino)-3-methylpentanamido)-5,5- dimethylhexanoic acid, resin bound **(Resin-21,** 39.0 mg, 0.08 mmol) was swollen in DMF at room temperature for 10 minutes and filtered. 20% Piperidine in DMF (5 mL) was added to the swollen resin, and nitrogen gas was passed through the suspension for 20 minutes. The suspension was filtered and 20% piperidine in DMF (5 mL) was added, and nitrogen gas was passed through the suspension for 20 minutes. The suspension was filtered and washed with DMF (5 mL x 3), DCM (5 mL x 3), water and DMF (5 mL x 3). The obtained solid was used without further purification. DMF (5 mL) was added to the obtained resin and pyrazine-2-carboxylic acid (**4**, 40.0 mg, 0.32 mmol), HATU (0.12 mg, 0.32 mmol) and 4-methylmorpholine (38.0 µL, 0.32 mmol) were added. Nitrogen gas was passed through the suspension for 2 hours. The suspension was filtered and washed with DMF (5 mL x 3), DCM (5 mL x 3), water (5 mL x 2) and DMF (5 mL x 3), and DMF (10 mL) was added. Pyrazine-2-carboxylic acid (4, 40.0 mg, 0.32 mmol), HATU (0.12 mg, 0.32 mmol) and 4-methylmorpholine (38.0 µL, 0.32 mmol) were added. Nitrogen gas was passed through for 15 hours. The reaction suspension was filtered and washed with DMF (5 mL x 3), DCM (5 mL x 3), water (5 mL x 2), DMF (5 mL x 2) and DCM (5 mL x 2), to afford resin bound intermediate **Resin-22** (39 mg, 0.030 mmol).

A mixture of TFA/water (95:5, 1 mL) was added to the resin bound intermediate **Resin-22** (39.0 mg, 0.08 mmol) and stirred at room temperature for 3 hours. The reaction mixture was filtered, and resin washed with DCM (20 mL x 3). The combined filtrates were concentrated *in vacuo* and azeotroped with toluene (15 mL x 3). The crude product was purified by preparative HPLC. This resulted in the title compound, (S)-5,5-dimethyl-2-((2S,3S)-3-methyl-2-(pyrazine-2-carboxamido) pentanamido) hexanoic acid **(Example 9,** 13.71 mg, 33 µmol, 41% yield, 90% Purity) as a white solid. 100% LCMS purity (Method 1, 1.39 min; M+H = 379.3); 90% NMR purity [¹H NMR (500 MHz, DMSO-d6) δ 12.55 (brs, 1H), 9.20 (d, J = 1.5 Hz, 1H), 8.91 (d, J = 2.5 Hz, 1H), 8.79 - 8.75 (m, 1H), 8.47 (d, J = 9.3 Hz, 1H), 8.43 (d, J = 7.6 Hz, 1H), 4.54 (dd, J = 9.3, 7.0 Hz, 1H), 4.14 (td, J = 8.0, 5.0 Hz, 1H), 1.93 - 1.85 (m, 1H), 1.70 - 1.45 (m, 3H), 1.24 - 1.05 (m, 3H), 0.92 (d, J = 6.8 Hz, 3H), 0.86 - 0.80 (m, 12H), containing 0.5% TBME].

### Example 10

### (S)-2-((S)-2-Acetamido-3-(1-methyl-1H-imidazol-5-yl)propanamido)-5,5-dimethylhexanoic acid

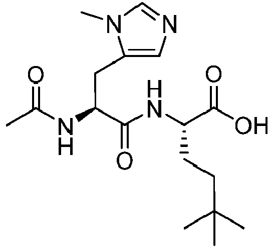

Example 10 was prepared in an analogous manner to Example 9.

(*S*)-2-((*S*)-2-Acetamido-3-(1-methyl-1*H*-imidazol-5-yl)propanamido)-5,5-dimethylhexanoic acid (4.4% yield, 97% LCMS purity). 97% LCMS purity (Method 1, 0.57 min; M+H = 353.3); 90 % NMR purity [¹H NMR (500 MHz, DMSO-d6) δ 12.64 (br s, 1H), 8.33 (d, J = 8.0 Hz, 1H), 8.10 (d, J = 8.6 Hz, 1H), 7.54 (s, 1H), 6.67 (s, 1H), 4.65 (dd, J = 8.2, 6.5 Hz, 1H), 4.11 (td, J = 8.2, 4.9 Hz, 1H), 3.57 (s, 3H), 2.86 (dd, J = 15.1, 6.5 Hz, 1H), 2.74 (dd, J = 15.1, 7.9 Hz, 1H), 1.81 (s, 3H), 1.65 - 1.55 (m, 1H), 1.52 - 1.43 (m, 1H), 1.09 - 1.05 (m, 2H), 0.82 (s, 9H), containing 4% IPA.].

### Example 11

### Synthesis of (S)-2-((S)-2-Acetamido-3-(1H-indol-3-yl)propanamido)-5,5-dimethylhexanoic acid

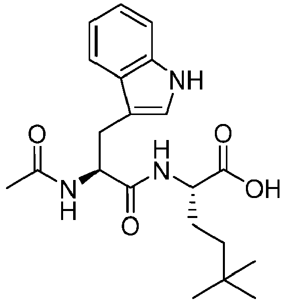

Example 11, which is the same compound as Example 6, was additionally prepared in an analogous manner to Example 9.

(*S*)-2-((*S*)-2-Acetamido-3-(1*H*-indol-3-yl)propanamido)-5,5-dimethylhexanoic acid (3.0% yield, 100% LCMS purity). 100% LCMS purity (Method 1, 1.18 min, M+H = 388.3). 98% NMR purity [¹H NMR (500 MHz, DMSO-d6) δ 10.79 - 10.75 (m, 1H), 8.12 (d, J = 8.2 Hz, 1H), 7.79 (s, 1H), 7.57 (d, J = 7.9 Hz, 1H), 7.31 (d, J = 8.1 Hz, 1H), 7.13 - 7.11 (m, 1H), 7.06 - 7.02 (m, 1H), 6.99 - 6.94 (m, 1H), 4.50 - 4.42 (m, 1H), 3.92 - 3.87 (m, 1H), 3.13 (dd, J = 14.7, 4.3 Hz, 1H), 2.86 (dd, J = 14.7, 9.7 Hz, 1H), 1.76 (s, 3H), 1.74 - 1.65 (m, 1H), 1.59 - 1.50 (m, 1H), 1.16 - 1.08 (m, 2H), 0.83 (s, 9H), containing 2% DCM, CO₂H peak not observed.]

### Example 12

### Synthesis of (S)-5,5-Dimethyl-2-((2S,3S)-3-methyl-2-((R)-morpholine-2-carboxamido)pentanamido)hexanoic acid

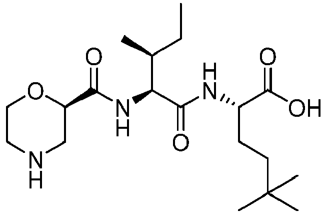

Example 12 was prepared in an analogous manner to Example 9.

(S)-5,5-Dimethyl-2-((2S,3S)-3-methyl-2-((R)-morpholine-2-carboxamido)pentanamido)hexanoic acid (3% yield, LCMS purity unconfirmed due to weak chromophore, Method 1, 0.67 min, M+H = 386.2). 97% NMR purity [¹H NMR (500 MHz, DMSO-d6) δ 8.03 (br s, 1H), 7.28 (d, J = 9.2 Hz, 1H), 4.25 - 4.18 (m, 1H), 4.03 - 3.94 (m, 1H), 3.88 - 3.82 (m, 2H), 3.56 - 3.46 (m, 1H), 2.99 - 2.93 (m, 1H), 2.72 - 2.58 (m, 2H), 2.48 - 2.42 (m, 1H), 1.78 - 1.72 (m, 1H), 1.70 -1.62 (m, 1H), 1.58-1.48 (m, 1H), 1.46-1.38 (m, 1H), 1.18-1.11 (m, 2H), 1.06 - 0.95 (m, 1H), 0.85 - 0.82 (m, 11H), 0.82 - 0.78 (m, 4H), NH and CO₂H peaks not observed.]

### Example 13

### Synthesis of (S)-5,5-Dimethyl-2-((2S,3S)-3-methyl-2-((S)-morpholine-2-carboxamido)pentanamido)hexanoic acid

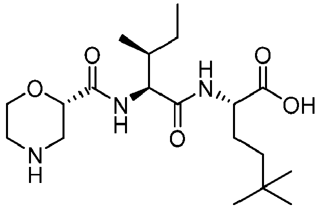

Example 13 was prepared in an analogous manner to Example 9.

(*S*)-5,5-Dimethyl-2-((2*S*,3*S*)-3-methyl-2-((*S*)-morpholine-2-carboxamido)pentanamido)hexanoic acid (20% yield, LCMS purity unconfirmed due to weak chromophore, Method 1, 0.66 min, M+H = 386.2). 95% NMR purity [¹H NMR (500 MHz, DMSO-d6) δ 9.72 - 8.66 (m, 1H), 8.09 (br s, 1H), 7.27 (d, J = 9.2 Hz, 1H), 4.26 (dd, J = 9.2, 6.9 Hz, 1H), 4.04 - 3.96 (m, 1H), 3.87 - 3.79 (m, 2H), 3.50 (td, J = 10.9, 3.2 Hz, 1H), 3.01 (dd, J = 12.5, 2.8 Hz, 1H), 2.71 - 2.62 (m, 2H), 2.48 - 2.41 (m, 1H), 1.78 - 1.70 (m, 1H), 1.69 - 1.61 (m, 1H), 1.58 - 1.49 (m, 1H), 1.45 - 1.36 (m, 1H), 1.18-1.11 (m, 2H), 1.05 - 0.95 (m, 1H), 0.83 (d, J = 2.0 Hz, 9H), 0.83 - 0.78 (m, 6H), containing 3% Acetonitrile, no CO₂H peak observed.]

### Example 14

### Synthesis of (S)-5,5-Dimethyl-2-((2S,3S)-3-methyl-2-((R)-morpholine-3-carboxamido)pentanamido)hexanoic acid

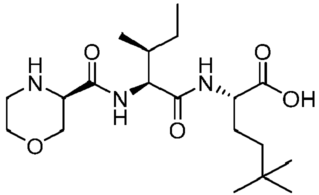

Example 14 was prepared in an analogous manner to Example 9.

(*S*)-5,5-Dimethyl-2-((2*S*,3*S*)-3-methyl-2-((*R*)-morpholine-3-carboxamido)pentanamido)hexanoic acid (7.2% yield, LCMS purity unconfirmed due to weak chromophore, Method 1, 0.67 min, M+H = 386.2). 95% NMR purity 95% [¹H NMR (500 MHz, DMSO-d6) δ 9.16 - 8.74 (m, 1H), 8.12 (br s, 1H), 7.25 (d, J = 9.2 Hz, 1H), 4.26 (dd, J = 9.2, 6.9 Hz, 1H), 4.04 - 3.97 (m, 1H), 3.86 - 3.78 (m, 2H), 3.50 (td, J = 10.9, 3.2 Hz, 1H), 3.01 (dd, J = 12.1, 2.8 Hz, 1H), 2.71 - 2.60 (m, 2H), 2.45 (dd, J = 12.4, 10.2 Hz, 1H), 1.77 - 1.69 (m, 1H), 1.69 - 1.61 (m, 1H), 1.60 - 1.49 (m, 1H), 1.44 - 1.36 (m, 1H), 1.18-1.11 (m, 2H), 1.05 - 0.95 (m, 1H), 0.83 (s, 9H), 0.83 - 0.79 (m, 6H), containing 2% Acetonitrile, CO₂H peak not observed.]

### Example 15

### Synthesis of (S)-5,5-Dimethyl-2-((2S,3S)-3-methyl-2-((S)-morpholine-3-carboxamido)pentanamido)hexanoic acid

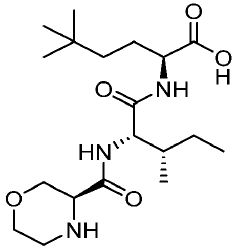

Example 15 was prepared in an analogous manner to Example 9.

(S)-5,5-Dimethyl-2-((2S,3S)-3-methyl-2-((S)-morpholine-3-carboxamido)pentanamido)hexanoic acid (15% yield, Method 1, 0.66 min, M+H = 386.2). 87% NMR purity [¹H NMR (500 MHz, DMSO-d6) δ 8.17 - 8.07 (m, 1H), 7.78 (d, J = 9.2 Hz, 1H), 4.27 (dd, J = 9.2, 6.6 Hz, 1H), 4.08 - 4.01 (m, 1H), 3.70 (dd, J = 10.9, 3.6 Hz, 1H), 3.55 (dt, J = 11.2, 3.7 Hz, 2H), 3.46 (dd, J = 10.9, 7.7 Hz, 1H), 2.91 - 2.82 (m, 1H), 2.78 - 2.61 (m, 3H), 1.79 - 1.71 (m, 1H), 1.71 - 1.61 (m, 1H), 1.61 - 1.49 (m, 1H), 1.49-1.39 (m, 1H), 1.22 - 1.11 (m, 2H), 1.08 - 0.99 (m, 1H), 0.85 (s, 2H), 0.84 (s, 9H), 0.82 (t, J = 7.9 Hz, 4H), CO₂H peak not observed.]

### Example 16

### Synthesis of (S)-2-((2S,3S)-2-Acetamido-3-methylpentanamido)-5,5-dimethylhexanoic acid

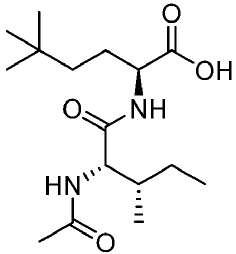

Example 16 was prepared in an analogous manner to Example 9.

(S)-2-((2S,3S)-2-Acetamido-3-methylpentanamido)-5,5-dimethylhexanoic acid (30% yield, 100% LCMS purity =, Method 3, 1.09 min, M+H = 315.2). 98% NMR purity [¹H NMR (500 MHz, DMSO-d6) δ 12.46 (br s, 1H), 8.06 (d, J = 7.3 Hz, 1H), 7.85 (d, J = 9.1 Hz, 1H), 4.23 (dd, J = 9.0, 7.5 Hz, 1H), 4.12 - 4.03 (m, 1H), 1.84 (s, 3H), 1.74 - 1.62 (m, 2H), 1.61 - 1.50 (m, 1H), 1.47- 1.38 (m, 1H), 1.25 - 1.13 (m, 2H), 1.13 - 1.02 (m, 1H), 0.85 (s, 9H), 0.83 (s, 3H), 0.80 (t, J = 7.4 Hz, 3H).]

### Example 17

### (2S)-2-[(2S)-3-(1-tert-butyl-1H-indol-3-yl)-2-acetamidopropanamido]-5,5-dimethylhexanoic acid

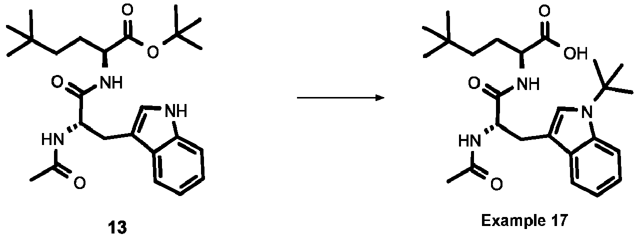

*Tert-*butyl (*S*)-2-((*S*)-2-acetamido-3-(1H-indol-3-yl)propanamido)-5,5-dimethylhexanoate **13** (480 mg, 1.08 mmol) was dissolved in ice/cold 2,2,2-trifluoroacetic acid (50 mL), the ice bath removed and stirred for 45 minutes. The TFA was evaporated in vacuo at 25 °C, azeotroped with toluene (50 mL) and then dissolved in DCM (10 mL) and adsorbed onto celite. The DCM was evaporated in vacuo and the material purified by chromatography on RP Flash C18 (24 g cartridge, 5-50% MeCN/10 mM ammonium bicarbonate) to afford the title compound (2S)-2-[(2S)-3-(1-tert-butyl-1H-indol-3-yl)-2-acetamidopropanamido]-5,5-dimethylhexanoic acid **(Example 17,** 4 mg, 0.009 mmol, 0.8 %) as a pale brown solid. 98% purity LCMS (Method 1, 1.61 min; M+H = 444.2). ¹H NMR (500 MHz, DMSO-d6) δ 12.61 (s, 1H), 8.15 (d, J = 7.7 Hz, 1H), 8.03 (d, J = 8.3 Hz, 1H), 7.65 - 7.57 (m, 2H), 7.28 (s, 1H), 7.07 (ddd, J = 8.4, 6.9, 1.3 Hz, 1H), 6.99 (app. t, J = 7.4 Hz, 1H), 4.63 - 4.55 (m, 1H), 4.16 - 4.09 (m, 1H), 3.08 (dd, J = 14.7, 5.1 Hz, 1H), 2.84 (dd, J = 14.8, 8.8 Hz, 1H), 1.77 (s, 3H), 1.73 - 1.65 (m, 1H), 1.64 (s, 9H), 1.61 - 1.49 (m, 1H), 1.24 - 1.11 (m, 2H), 0.85 (s, 9H).

### Example 18

### (2S)-2-[(2S)-2-acetamido-3-(1-methyl-1H-indol-3-yl)propanamido]-5,5-dimethylhexanoic acid

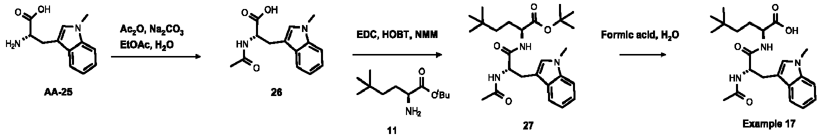

### Synthesis of (2S)-2-acetamido-3-(1-methyl-1H-indol-3-yl)propanoic acid (26)

Ac₂O (0.16 mL, 1.6 mmol) was added dropwise to a vigorously stirred mixture of 1-methyl-L-tryptophan (0.30 g, 1.4 mmol) and sodium carbonate (0.44 g, 4.1 mmol) in EtOAc (10 mL) and water (10 mL) and then stirred for 2 hours. The reaction was adjusted to pH 3 with 2M HCI, extracted with EtOAc (20 mL), washed with brine (10 mL), dried (MgSO₄), filtered and evaporated in vacuo to afford (2S)-2-acetamido-3-(1-methyl-1H-indol-3-yl)propanoic acid **(26,** 0.31 g, 1.1 mmol, 83 %) as an off-white solid. 97% purity LCMS (Method 1, 0.98 min; M+H = 261.1). ¹H NMR (500 MHz, DMSO-d6) δ 12.59 (s, 1H), 8.13 (d, J = 7.8 Hz, 1H), 7.54 (d, J = 7.8, Hz, 1H), 7.37 (d, J = 8.2 Hz, 1H), 7.13 (dd, J = 8.1, 6.9 Hz, 1H), 7.10 (s, 1H), 7.02 (dd, J = 8.0, 6.9 Hz, 1H), 4.44 (td, J = 8.2, 5.2 Hz, 1H), 3.72 (s, 3H), 3.13 (dd, J = 14.6, 5.3 Hz, 1H), 2.98 (dd, J = 14.6, 8.5 Hz, 1H), 1.80 (s, 3H).

### Synthesis of tert-butyl (2S)-2-((2S)-2-acetamido-3-(1-methyl-1H-indol-3-yl)propanamido)-5,5-dimethylhexanoate (27)

HOBt (97 mg, 0.63 mmol) was added to a stirred solution of Nα-acetyl-1-methyl-L-tryptophan (0.15 g, 0.58 mmol) and tert-butyl (2S)-2-amino-5,5-dimethylhexanoate (0.13 g, 0.61 mmol) in DMF (1 mL). The reaction was cooled to 0 °C, EDC (0.12 g, 0.61 mmol) and 4-methylmorpholine (0.13 mL, 1.2 mmol) added, allowed to attain room temperature and stirred for 4 h. The reaction was quenched with water (20 mL), extracted with EtOAc (2 x 20 mL), washed with water (2 x 10 mL) and brine (5mL), dried (MgSO₄), filtered, evaporated in vacuo and purified by chromatography on silica gel (24 g FlashPure column, 0 - 100% EtOAc/isohexane) to afford tert-butyl (2*S*)-2-((2*S*)-2-acetamido-3-(1-methyl-1H-indol-3-yl)propanamido)-5,5-dimethylhexanoate **(27,** 0.17 g, 0.36 mmol, 63 %) as a colourless gum. 100% purity LCMS (Method 2, 1.83 min; M+H = 458.1). ¹H NMR (500 MHz, DMSO-d6) δ 8.23 (d, J = 7.4 Hz, 1H), 8.02 (d, J = 8.3 Hz, 1H), 7.66 - 7.62 (m, 1H), 7.37 - 7.34 (m, 1H), 7.14 - 7.10 (m, 1H), 7.09 (s, 1H), 7.03 - 6.99 (m, 1H), 4.58 (td, J = 8.8, 4.6 Hz, 1H), 4.08 (td, J = 7.4, 5.7 Hz, 1H), 3.72 (s, 3H), 3.09 (dd, J = 14.6, 4.6 Hz, 1H), 2.87 (dd, J = 14.7, 9.3 Hz, 1H), 1.76 (s, 3H), 1.69 - 1.52 (m, 2H), 1.40 (s, 9H), 1.21 - 1.16 (m, 2H), 0.86 (s, 9H).

### Synthesis of (S)-2-((S)-2-acetamido-3-(1-methyl-1H-indol-3-yl)propanamido)-5,5-dimethylhexanoic acid (Example 18)

*tert*-butyl (2*S*)-2-((2*S*)-2-acetamido-3-(1-methyl-1H-indol-3-yl)propanamido)-5,5-dimethylhexanoate (165 mg, 361 µmol) was dissolved in an ice/cold mixture of formic acid (5 mL) and water (0.5 mL). The ice bath was then removed and the reaction stirred for 20 hours. The solvent was evaporated in vacuo, azeotroped with toluene and treated with ether to afford the title compound (2S)-2-((2S)-2-acetamido-3-(1-methyl-1H-indol-3-yl)propanamido)-5,5-dimethylhexanoic acid **(Example 18,** 142 mg, 0.35 mmol, 96 %) as an off-white solid. 98% purity LCMS (Method 1, 1.39 min; M+H = 402.5). ¹H NMR (500 MHz, DMSO-d6) δ 12.60 (s, 1H), 8.17 (d, J = 7.7 Hz, 1H), 8.02 (d, J = 8.3 Hz, 1H), 7.62 (d, J = 7.9 Hz, 1H), 7.35 (d, J = 8.2 Hz, 1H), 7.12 (t, J = 7.6 Hz, 1H), 7.09 (s, 1H), 7.01 (t, J = 7.4 Hz, 1H), 4.57 (td, J = 8.6, 4.8 Hz, 1H), 4.16 (td, J = 8.1, 5.3 Hz, 1H), 3.71 (s, 3H), 3.09 (dd, J = 14.7, 4.8 Hz, 1H), 2.87 (dd, J = 14.7, 9.0 Hz, 1H), 1.76 (s, 3H), 1.73 - 1.65 (m, 1H), 1.63 - 1.53 (m, 1H), 1.26 - 1.11 (m, 2H), 0.86 (s, 9H).

### BIOLOGICAL DATA

### Neurotensin scintillation proximity assay

The data of exemplified compounds of the invention tested in a Neurotensin (NTS) scintillation proximity assay (SPA). The IC₅₀ data is shown in the table below. NTS, which is a 13 amino acid neuropeptide, is a sortilin ligand. The IC50 is a measure of the amount of the compound required to inhibit the binding of NTS to sortilin by 50%. The skilled person will recognise that the lower the IC₅₀ value, the less of the compound needed to achieve the desired effect, and as a result, the chances of undesirable off-target effects are reduced.

Compound affinity was determined by measuring the displacement of [3H]-neurotensin or [125I]-neurotensin binding to hSortilin in SPA format. Total volume of 40 µl in 50 mM HEPES pH 7.4 assay buffer containing 100 mM NaCl, 2.0 mM CaCI2, 0.1% BSA and 0.1% Tween-20. Compound pre-incubation for 30 minutes at room temperature with 150 nM of 6his-Sortilin before 5 nM [3H]-Neurotensin or [125I]-neurotensin and Ni chelate imaging beads (Perkin Elmer) were added, after 6 hours plate was read on a ViewLux with 360 s exposure time. Dose-response evaluation of compounds was performed with 8 concentrations of drugs (covering 3 decades). IC₅₀ values were calculated by nonlinear regression using the sigmoid concentration-response (variable slope) using CDD Vault software. All values reported are average of at least 2 determinations.

The data in the table below shows that the compounds disclosed herein are sortilin inhibitors.

| **Representative Examples** | **IC₅₀ [3H]Neurotensin SPA** | **IC₅₀ [125I]Neurotensin SPA** |
|---|---|---|
| Example 1 | 1670 nM | - |
| Example 3 | 90 nM | - |
| Example 4 | 140 nM | - |
| Example 5 | 270 nM | - |
| Examples 6 and 11 | 60 nM | - |
| Example 8 | - | 2.17 µM |
| Example 9 | - | 620 nM |
| Example 10 | 2.03 µM | - |
| Example 12 | 150 nM | |
| Example 13 | 120 nM | - |
| Example 14 | 240 nM | - |
| Example 15 | 310 nM | - |
| Example 16 | - | 290 nM |
| Example 17 | 100 nM | - |
| Example 18 | 90 nM | - |

### X-RAY DATA

### Materials and methods

sSortilin, the luminal domain of sortilin, was obtained as previously described (Andersen et al., Acta Cryst. D, 2017)¹⁷. Prior to crystallization 12 µl of 4 mg/ml sSortilin in 50 mM Tris-HCI pH 8.0, 150 mM NaCl was mixed with 1.2 µl of the two compounds INS1767 and INS1783 dissolved in DMSO at concentrations of 16.7 mM and 13.2 mM respectively.

Sitting drops were set up by adding 2 µl of the sortilin ligand mixture to 2 µl of reservoir solution composed of 100 mM Hepes pH 7.3, 400 mM malonate pH 7.3, 8% v/v glycerol, 22.5% w/v PEG3350. The sitting drops were left to equilibrate by vapor diffusion with 500 µl reservoir solution.

Crystals were mounted in litho-loops without further cryoprotection and were flash cooled in liquid nitrogen.

Diffraction data were collected at beamline P13 EMBL/DESY, Hamburg, and processed using the XDS package (Kabsch, W., Acta Cryst. D, 2010)¹⁸ (Table 1) Phases for the structure factors were obtained by molecular replacement using the known structure of sortilin (PDB entry: 3F6K) as search model and the program Phaser implemented in the Phenix software package (Afonine et al., Acta Cryst. D, 2012)¹⁹. A refined model was obtained by several cycles of model building in Coot (Emsley P. et al., Acta Cryst. D, 2010)²⁰ and maximum likelihood refinement using Phenix. The resulting X-ray derived picture of the compound of Example 3 bound to *h*-sortilin is shown in Figure 1. Refinement statistics and agreement of the models with standard geometry are shown in the table below.

### Refinement statistics and agreement of the models with standard geometry

### (Statistics for the highest resolution shell are shown in parentheses)

| | **Example 3** |
|---|---|
| Wavelength | |
| Resolution range | 47.2 - 2.6 (2.69 -2.6) |
| Space group | C 1 2 1 |
| Unit cell (a,b,c) Å | 162.54 78.24 112.5 |
| (α,β,γ) ° | 90 126.951 90 |
| Total reflections | 462840 (44942) |
| Unique reflections | 34897 (3450) |
| Multiplicity | 13.3 (13.0) |
| Completeness (%) | 99.89 (99.83) |
| Mean I/sigma(I) | 15.36 (1.17) |
| Wilson B-factor | 86.37 |
| R-merge | 0.09716 (2.081) |
| R-meas | 0.1012 (2.166) |
| R-pim | 0.02794 (0.5955) |
| CC1/2 | 0.999 (0.645) |
| CC* | 1 (0.886) |
| Reflections in refinement | 34878 (3445) |
| Reflections used for R-free | 1394 (138) |
| R-work | 0.2017 (0.4356) |
| R-free | 0.2343 (0.4964) |
| CC(work) | 0.954 (0.761) |
| CC(free) | 0.933 (0.747) |
| Number of non-hydrogen atoms macromolecules | 5396 |
| Protein residues | 657 |
| RMS(bonds) Å | 0.002 |
| RMS(angles) ° | 0.42 |
| Ramachandran favored (%) | 95.39 |
| Ramachandran allowed (%) | 4.30 |
| Ramachandran outliers (%) | 0.31 |
| Rotamer outliers (%) | 1.97 |
| Clashscore | 3.97 |
| Average B-factor macromolecules | 107.08 |
| Number of TLS groups | 6 |

### REFERENCES

1. Tauris, J., et al., Proneurotrophin-3 May Induce Sortilin-Dependent Death In Inner Ear Neurons. Eur J Neuroscience (2020), 33(4), pp.622-31.
2. Goettsch, C., et al., Sortilin and Its Multiple Roles in Cardiovascular and Metabolic Diseases. Atherosclerosis, Thrombosis and Vascular Biology (2017), 38(1), pp. 19-25.
3. Willnow, T.E., et al., Sortilins: new players in lipoprotein metabolism. Current Opinion in Lipidology (2011), 22(2), pp. 79-85.
4. Kjolby, M., et al., Sort1, encoded by the cardiovascular risk locus 1p13.3, is a regulator of hepatic lipoprotein export. Cell Metabolism (2010), 12(3), pp. 213-223.
5. Jansen, P., et al., Roles for the pro-neurotrophin receptor sortilin in neuronal development, aging and brain injury. Nature Neuroscience (2007), 10(11), pp.1449-1457.
6. Tenk, H.K., et al., ProBDNF induces neuronal apoptosis via activation of a receptor complex of p75NTR and sortilin. J Neuroscience (2005), 10(11), pp.1449-1457.
7. Nykjaer, A., et al., Sortilin is essential for proNGF-induced neuronal cell death. Nature (2004), 427(6977), pp.843-848.
8. Huang, G. et al., Insulin responsiveness of glucose transporter 4 in 3T3-L1 cells depends on the presence of sortilin. Mol Biol Cell (2013), 24(19), pp.3115-3122.
9. Pan, X. et al., Sortilin and retromer mediate retrograde transport of Glut4 in 3T3-L1 adipocytes. Mol Biol Cell (2017), 28(12), pp.1667-1675.
10. Kaddai, V. et al. Involvement of TNF-α in abnormal adipocyte and muscle sortilin expression in obese mice and humans. Diabetologia (2009) 52, pp. 932-940.
11. Mortensen, M.B. et al., Targeting sortilin in immune cells reduces proinflammatory cytokines and atherosclerosis. J Clin Invest (2014), 124(12), pp. 5317-5322.
12. Shi, J. & Kandror, K. V., Sortilin Is Essential and Sufficient for the Formation of Glut4 Storage Vesicles in 3T3-L1 Adipocytes. Developmental Cell (2005), 9, pp. 99-108.
13. Gao, A. et al., Implications of Sortilin in Lipid Metabolism and Lipid Disorder Diseases. DNA and Cell Biology (2017), 36(12), pp.1050-1061.
14. Oh, T.J. et al., Circulating sortilin level as a potential biomarker for coronary atherosclerosis and diabetes mellitus. Cardiovascular Diabetology (2017), 16(92).
15. Skeldal, S. et al., Mapping of the Interaction Site between Sortilin and the p75 Neurotrophin Receptor Reveals a Regulatory Role for the Sortilin Intracellular Domain in p75 Neurotrophin Receptor Shedding and Apoptosis. J Biol Chem (2012), 21(287), pp. 43798-43809.
16. Wuts, P.G.M. and Greene, T.W, Greene's Protective Groups in Organic Synthesis, 4th Edition, John Wiley and Sons, New York (2006).
17. Andersen, K R et al., Introducing site-specific cysteines into nanobodies for mercury labelling allows de novo phasing of their crystal structures. Acta Crystallographia Section. D (2017),73(1), pp. 804-813.
18. Kabsch, W, XDS. Acta Crystallographia Section. D (2010), 66(2), pp. 125-132.
19. Afonine, P V et al., Acta Crystallographia Section. D (2012),68(4), pp. 352-367.
20. Emsley, P. et al., Acta Crystallographia Section. D (2010),66(4), pp. 486-501.

### Sequences referenced throughout the specification and forming part of the description

SEQ ID NO: 1 (full length sortilin- isoform 1) 
1 
51 
101 
151 
201 
251 
301 
351 
401 
451 
501 
551 
601 
651 
701 
751 
801 
SEQ ID NO: 2 (full length sortilin- isoform 2) 
1 MERPWGAADG LSRWPHGLGL LLLLQLLPPS TLSQDRLDAP PPPAAPLPRW
51 SGPIGVSWGL RAAAAGGAFP RGGRWRRSAP GEDEECGRVR DFVAKLANNT
101 HQHVFDDLRG SVSLSWVGDS TGVILVLTTF HVPLVIMTFG QSKLYRSEDY
151 GKNFKDITDL INNTFIRTEF GMAIGPENSG KVVLTAEVSG GSRGGRIFRS
201 SDFAKNFVQT DLPFHPLTQM MYSPQNSDYL LALSTENGLW VSKNFGGKWE
251 EIHKAVCLAK WGSDNTIFFT TYANGSCTDL GALELWRTSD LGKSFKTIGV
301 KIYSFGLGGR FLFASVMADK DTTRRIHVST DQGDTWSMAQ LPSVGQEQFY
351 SILAANDDMV FMHVDEPGDT GFGTIFTSDD RGIVYSKSLD RHLYTTTGGE
401 TDFTNVTSLR GVYITSVLSE DNSIQTMITF DQGGRWTHLR KPENSECDAT
451 AKNKNECSLH IHASYSISQK LNVPMAPLSE PNAVGIVIAH GSVGDAISVM
501 VPDVYISDDG GYSWTKMLEG PHYYTILDSG GIIVAIEHSS RPINVIKFST
551 DEGQCWQTYT FTRDPIYFTG LASEPGARSM NISIWGFTES FLTSQWVSYT
601 IDFKDILERN CEEKDYTIWL AHSTDPEDYE DGCILGYKEQ FLRLRKSSVC
651 QNGRDYVVTK QPSICLCSLE DFLCDFGYYR PENDSKCVEQ PELKGHDLEF
701 CLYGREEHLT TNGYRKIPGD KCQGGVNPVR EVKDLKKKCT SNFLSPEKQN
751 SKSNSVPIIL AIVGLMLVTV VAGVLIVKKY VCGGRFLVHR YSVLQQHAEA
801 NGVDGVDALD TASHTNKSGY HDDSDEDLLE
SEQ ID NO: 3 (mature sortilin)

1 MTFGQSKLYR SEDYGKNFKD ITDLINNTFI RTEFGMAIGP ENSGKVVLTA
51 EVSGGSRGGR IFRSSDFAKN FVQTDLPFHP LTQMMYSPQN SDYLLALSTE
101 NGLWVSKNFG GKWEEIHKAV CLAKWGSDNT IFFTTYANGS CTDLGALELW
151 RTSDLGKSFK TIGVKIYSFG LGGRFLFASV MADKDTTRRI HVSTDQGDTW
201 SMAQLPSVGQ EQFYSILAAN DDMVFMHVDE PGDTGFGTIF TSDDRGIVYS
251 KSLDRHLYTT TGGETDFTNV TSLRGVYITS VLSEDNSIQT MITFDQGGRW
301 THLRKPENSE CDATAKNKNE CSLHIHASYS ISQKLNVPMA PLSEPNAVGI
351 VIAHGSVGDA ISVMVPDVYI SDDGGYSWTK MLEGPHYYTI LDSGGIIVAI
401 EHSSRPINVI KFSTDEGQCW QTYTFTRDPI YFTGLASEPG ARSMNISIWG
451 FTESFLTSQW VSYTIDFKDI LERNCEEKDY TIWLAHSTDP EDYEDGCILG
501 YKEQFLRLRK SSVCQNGRDY VVTKQPSICL CSLEDFLCDF GYYRPENDSK

551 CVEQPELKGH DLEFCLYGRE EHLTTNGYRK IPGDKCQGGV NPVREVKDLK
601 KKCTSNFLSP EKQNSKSNSV PIILAIVGLM LVTVVAGVLI VKKYVCGGRF
651 LVHRYSVLQQ HAEANGVDGV DALDTASHTN KSGYHDDSDE DLLE

## Claims

1. A compound of formula (I) 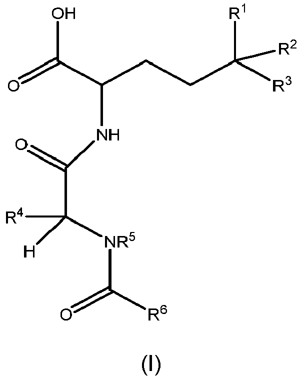 or a pharmaceutically acceptable salt, solvate, hydrate, tautomer, optical isomer, N-oxide, and/or prodrug thereof; wherein
R¹, R² and R³ are each independently selected from the group consisting of halo, H, (C₁-C₄)alkyl, halo-(C₁-C₄)alkyl, (C₂-C₄)alkenyl, and halo-(C₂-C₄)alkenyl;
R⁴ is selected from the group consisting of H, (C₁-C₁₀)alkyl, halo-(C₁-C₁₀)alkyl, (C₂-C₁₀)alkenyl, halo-(C₂-C₁₀)alkenyl, (C₃-C₂₀)aryl, halo-(C₃-C₂₀)aryl, (C₃-C₈)heteroaryl, halo-(C₃-C₂₀)heteroaryl, (C₁-C₆)-alkylene-(C₃-C₂₀)-aryl, (C₁-C₆)-alkylene-(C₃-C₂₀)-heteroaryl, (C₁-C₆)-alkylene-(3- to 10- membered-heterocyclic ring);
wherein the aryl group in (C₁-C₆)-alkylene-(C₃-C₂₀)-aryl, the heteroaryl group in (C₁-C₆)-alkylene-(C₃-C₂₀)-heteroaryl or the heterocyclic ring in (C₁-C₆)-alkylene-(3- to 8- membered heterocyclic ring) is optionally substituted with one or more substituents independently selected from halo, H, -OH, (C₁-C₄)alkyl, halo-(C₁-C₄)alkyl, (C₁-C₄)alkoxy and halo-(C₁-C₄)alkoxy;
R⁵ is selected from the group consisting of H, (C₁-C₄)alkyl, halo-(C₁-C₄)alkyl, (C₂-C₄)alkenyl and halo-(C₁-C₄)alkenyl; and
R⁶ is selected from the group consisting of (C₁-C₆)alkyl, halo-(C₁-C₆)alkyl, (C₂-C₆)alkenyl, halo-(C₂-C₆)alkenyl, (C₃-C₂₀)aryl, halo-(C₃-C₂₀)aryl, (C₃-C₂₀)heteroaryl and halo-(C₃-C₂₀)heteroaryl.

2. The compound according to claim 1, wherein R¹, R² and R³ are each independently selected from the group consisting of halo, (C₁-C₂)alkyl and halo-(C₁-C₂)alkyl.

3. The compound according to claim 1 or claim 2, wherein R¹, R² and R³ are each independently selected from F, CH₃ and CF₃.

4. The compound according to any preceding claim, wherein R⁴ is selected from the group consisting of H, (C₁-C₆)alkyl, halo-(C₁-C₆)alkyl, (C₃-C₁₀)aryl, (C₁-C₃)-alkylene-(C₃-C₁₀)-aryl, (C₁-C₃)-alkylene-(C₃-C₂₀)-heteroaryl and (C₁-C₃)-alkylene-(3- to 10- membered-heterocyclic ring);
wherein the aryl group in (C₁-C₃)-alkylene-(C₃-C₁₀)-aryl, the heteroaryl group in (C₁-C₃)-alkylene-(C₃-C₁₀)-heteroaryl or the heterocyclic ring in (C₁-C₃)-alkylene-(3- to 8- membered heterocyclic ring) is optionally substituted with one or more substituents independently selected from halo, -OH, (C₁-C₄)alkyl, halo-(C₁-C₄)alkyl, (C₁-C₄)alkoxy and halo-(C₁-C₄)alkoxy.

5. The compound according to any preceding claim, wherein R⁴ is selected from the group consisting of:
(i) H
(ii) CH₃

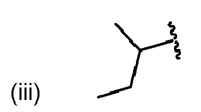

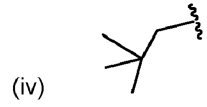

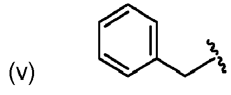

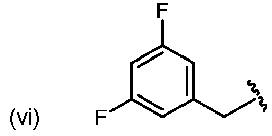

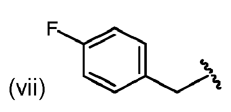

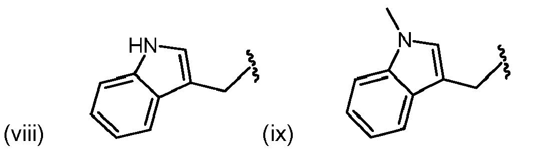

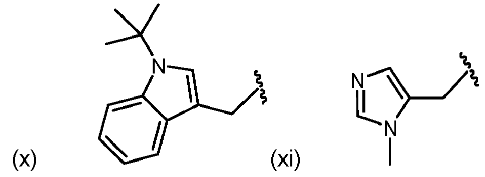

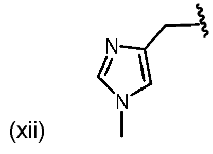

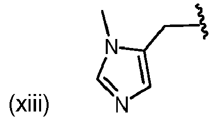

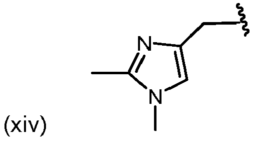

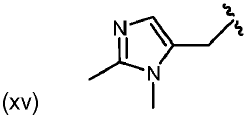

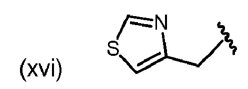

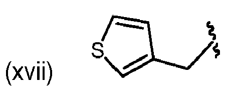

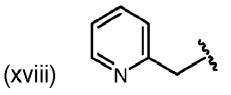

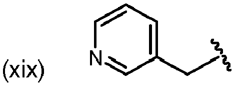

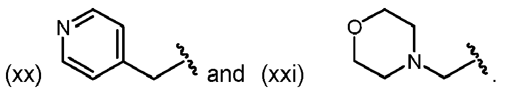

6. The compound according to any preceding claim, wherein R⁵ is selected from the group consisting of H, (C₁-C₃)-alkyl and (C₁-C₃)haloalkyl.

7. The compound according to any preceding claim, wherein R⁵ is selected from the group consisting of H and CH₃.

8. The compound according to any preceding claim, wherein R⁶ is selected from the group consisting of (C₁-C₄)alkyl, halo-(C₁-C₄)alkyl, (C₃-C₈)heteroaryl, and halo-(C₃-C₈)heteroaryl.

9. The compound according to any preceding claim, wherein R⁶ is selected from the group consisting of CH₃, pyrazine and morpholine.

10. The compound according to any preceding claim, wherein the compound of Formula (I) is:
(S)-2-(2-acetamidoacetamido)-5,5-dimethylhexanoic acid;
(S)-5,5-dimethyl-2-(2-(pyrazine-2-carboxamido)acetamido)hexanoic acid;
(S)-2-((S)-2-acetamido-3-phenylpropanamido)-5,5-dimethylhexanoic acid;
(2S)-5,5-dimethyl-2-{2-[(pyrazin-2-yl)formamido]acetamido}hexanoic acid;
(2S)-2-[(2S,3S)-2-acetamido-3-methylpentanamido]-5,5-dimethylhexanoic acid;
(2S)-5,5-dimethyl-2-[(2S,3S)-3-methyl-2-[(pyrazin-2-yl)formamido]pentanamido]hexanoic acid;
(2S)-5,5,5-trifluoro-2-[(2S,3S)-3-methyl-2-[(pyrazin-2-yl)formamido]pentanamido]pentanoic acid;
(2S)-2-[(2S)-2-acetamido-3-phenylpropanamido]-5,5-dimethylhexanoic acid;
(2S)-2-[(2S)-2-acetamido-3-(4-fluorophenyl)propanamido]-5,5-dimethylhexanoic acid;
(2S)-2-[(2S)-3-(3,5-difluorophenyl)-2-acetamidopropanamido]-5,5-dimethylhexanoic acid;
(2S)-2-[(2S)-2-acetamido-3-(1H-indol-3-yl)propanamido]-5,5-dimethylhexanoic acid;
(2S)-2-[(2S)-2-acetamido-3-(1-methyl-1H-imidazol-5-yl)propanamido]-5,5-dimethylhexanoic acid;
(S)-5,5-dimethyl-2-((S)-3-phenyl-2-(pyrazine-2-carboxamido)propanamido)hexanoic acid;
(2S)-2-[(2S)-2-acetamido-3-(1-methyl-1H-imidazol-4-yl)propanamido]-5,5-dimethylhexanoic acid;
(2S)-5,5-dimethyl-2-[(2S,3S)-3-methyl-2-{[(3S)-morpholin-3-yl]formamido}pentanamido]hexanoic acid;
(2S)-5,5-dimethyl-2-[(2S,3S)-3-methyl-2-{[(3R)-morpholin-3-yl]formamido}pentanamido]hexanoic acid;
(2S)-5,5-dimethyl-2-[(2S,3S)-3-methyl-2-{[(2S)-morpholin-2-yl]formamido}pentanamido]hexanoic acid;
(2S)-5,5-dimethyl-2-[(2S,3S)-3-methyl-2-{[(2R)-morpholin-2-yl]formamido}pentanamido]hexanoic acid;
(2S)-5,5-dimethyl-2-[(2S)-3-(1-methyl-1H-imidazol-4-yl)-2-(N-methylacetamido)propanamido]hexanoic acid;
(2S)-5,5-dimethyl-2-[(2S)-3-(1-methyl-1H-imidazol-5-yl)-2-(N-methylacetamido)propanamido]hexanoic acid;
(2S)-5,5-dimethyl-2-[(2S)-3-(1-methyl-1H-imidazol-4-yl)-2-(2-oxopyrrolidin-1-yl)propanamido]hexanoic acid;
(2S)-2-[(2S)-3-(1,2-dimethyl-1H-imidazol-5-yl)-2-(N-methylacetamido)propanamido]-5,5-dimethylhexanoicacid;
(2S)-2-[(2S)-3-(1,2-dimethyl-1H-imidazol-4-yl)-2-(N-methylacetamido)propanamido]-5,5-dimethylhexanoicacid;
(2S)-2-[(2R)-2-acetamido-3-(1H-indol-3-yl)propanamido]-5,5-dimethylhexanoic acid;
(2S) - 2 - [(2S) - 2 - acetamido - 3 - (1,3 - thiazol - 4 - yl)propanamido] - 5,5 - dimethylhexanoic acid;
(2S) - 2 - [(2S) - 2 - acetamidopropanamido] - 5,5 - dimethylhexanoic acid;
(2S) - 2 - [(2S) - 2 - acetamido - 3 - (thiophen - 3 - yl)propanamido] - 5,5 - dimethylhexanoic acid;
(2S) - 2 - [(2S) - 2 - acetamido - 3 - (pyridin - 2 - yl)propanamido] - 5,5 - dimethylhexanoic acid;
(2S) - 2 - [(2S) - 2 - acetamido - 3 - (pyridin - 3 - yl)propanamido] - 5,5 - dimethylhexanoic acid;
(2S) - 2 - [(2S) - 2 - acetamido - 3 - (pyridin - 4 - yl)propanamido] - 5,5 - dimethylhexanoic acid;
(2S) - 2 - [(2S) - 2 - acetamido - 3 - (morpholin - 4 - yl)propanamido] - 5,5 - dimethylhexanoic acid; and
(2S)-2-[(2S)-2-acetamido-3-(1-methyl-1H-indol-3-yl)propanamido] - 5,5 - dimethylhexanoic acid.

11. A pharmaceutical composition comprising a compound according to any preceding claim and a pharmaceutically acceptable carrier, excipient, and/or diluent.

12. The compound according to any one of claims 1 to 10, or the pharmaceutical composition of claim 10, for use in therapy.

13. The compound according to any one of claims 1 to 9, or the pharmaceutical composition of claim 10, for use in the treatment or prevention of a neurodegenerative disorder, an inflammatory disorder, a cancer, pain, diabetes mellitus, diabetic retinopathy, glaucoma, uveitis, cardiovascular disease, hereditary eye conditions or hearing loss.

14. The compound or pharmaceutical composition for use according to claim 12,
wherein the neurodegenerative disorder is selected from frontotemporal dementia, Alzheimer's disease, Parkinson's disease and spinal cord injury;
wherein the inflammatory disorder may be selected from inflammatory diseases and neuroinflammation;
wherein the cancer is selected from breast cancer, lung cancer, ovarian cancer, prostate cancer, thyroid cancer, pancreatic cancer, glioblastoma and colorectal cancer; and
wherein the hearing loss is selected from noise-induced hearing loss, ototoxicity induced hearing loss, age-induced hearing loss, idiopathic hearing loss, tinnitus and sudden hearing loss.
